(19) 

Europäisches
Patentamt
European
Patent Office
Office européen
des brevets

(11) **EP 1 884 193 A1**

(12) **EUROPEAN PATENT APPLICATION**
published in accordance with Art. 153(4) EPC

(43) Date of publication:
06.02.2008 Bulletin 2008/06

(21) Application number: 06746109.5

(22) Date of filing: 09.05.2006

(51) Int Cl.:
*A61B 6/00* (2006.01)     *A61B 5/00* (2006.01)
*A61B 6/03* (2006.01)     *G06T 1/00* (2006.01)

(86) International application number:
**PCT/JP2006/309278**

(87) International publication number:
**WO 2006/126384 (30.11.2006 Gazette 2006/48)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: 23.05.2005  JP 2005149298
02.06.2005  JP 2005162444
08.06.2005  JP 2005168160
15.06.2005  JP 2005175015

(71) Applicant: **Konica Minolta Medical & Graphic, Inc.
Tokyo 163-0512 (JP)**

(72) Inventors:
• **KASAI, Satoshi**
c/o Konica Minolta Medical & Graphic
Tokyo 1630512 (JP)
• **INENAGA, Yoriko**
c/o Konica Minolta Medical & Graphic
Tokyo 1630512 (JP)
• **MATSUI, Ko**
c/o Konica Minolta Medical & Graphic
Tokyo 1630512 (JP)

(74) Representative: **Henkel, Feiler & Hänzel
Patentanwälte
Maximiliansplatz 21
80333 München (DE)**

(54) **ABNORMAL SHADOW CANDIDATE DISPLAY METHOD, AND MEDICAL IMAGE PROCESSING SYSTEM**

(57)     In medical image processing system 100, when a medical image is generated, detection processing of abnormal shadow candidate is applied to the medical image in image processing apparatus 2. Next, based on image characteristic amount of the detected abnormal shadow candidate, clearly truly positive or falsely positive abnormal shadow candidate, a candidate wherein it is difficult to determine whether it is truly positive or falsely positive and a candidate in a position difficult to detect are determined by multivariable analysis, and then deletion from the detected abnormal shadow candidates is conducted. Only the detection information of the abnormal shadow candidate after the deletion is displayed on viewer 5.

FIG. 1

100

IMAGE GENERATING APPARATUS — 1
IMAGE SERVER — 4
IMAGE DB — 4a
VIEWER — 5
IMAGE PROCESSING APPARATUS — 2
PRINTER — 3
N

**EP 1 884 193 A1**

**Description**

**FIELD OF THE INVENTION**

The present invention relates to an abnormal shadow candidate display method and a medical image processing system to detect an abnormal shadow candidate from a medical image and to display the detected information.

**BACKGROUND OF THE INVENTION**

In the field of medical treatment, when a medical image of a patient captured by an radiographing apparatus such as a CT (Computed Tomography) and MRI (Magnetic Resonance Imaging) is converted into digital data and is diagnosed by a doctor, the image has come to be shown on a display for radiographic interpretation. Particularly in recent years, a Computer Aided Diagnosis System (hereinafter referred to as "CAD") for detecting an abnormal shadow candidate at the cancer-affected region has been developed to reduce the load on the doctor interpreting a radiograph and to minimize the possibility of an abnormal shadow being overlooked.

When an abnormal shadow candidate has been detected by this CAD, a marker (an arrow or a circle) for indicating the position of the detected abnormal shadow candidate is generally displayed on the medical image as the detection information. The display method is available in two types: (1) a method wherein the marker is directly displayed on the medical image (for example, see Patent Document 1), and (2) a method wherein a life-size medical image (100% rate) for radiographic interpretation is displayed as the main image; and a sub-image is created wherein a marker is indicated on the reduced image of the main image, and is used together with the main image (for example, see Patent Document 2).

Patent Document 1: Unexamined Japanese Patent Application Publication No. 2000-276587
Patent Document 2: Unexamined Japanese Patent Application Publication No. 2004-230001

**DISCLOSURE OF THE INVENTION**

**PROBLEMS TO BE SOLVED BY THE INVENTION**

In the aforementioned arrangements, when there are a great number of abnormal shadow candidates detected by the CAD, a great number of markers are indicated on an image to be examined by a doctor in the aforementioned method (1). This makes it difficult to read the medical image, and adversely affects the process of radiographic interpretation.

In the meantime, as compared with the method (1), the method (2) gives less adverse affect the radiographic interpretation by doctor because the main image per se contains no marker, but a great number of markers appear on the reduced sub-image. This makes it difficult to examine the image. Further to this problem, it is also difficult to understand the positional relationship between the main and sub-images.

In a further method, when an abnormal shadow candidate is detected, the characteristic amount of the image in the candidate region is calculated, and comparison is made with a threshold value, thereby determining if there is any abnormal shadow or not. In a still further method, the candidate region of an abnormal shadow is detected by multivariable analysis using the characteristic amount of the image. As shown in the aforementioned examples of methods, various forms of algorithm are employed. However, the result of detection contains both clearly abnormal shadows and dubious ones in a mixed form.

In the conventional CAD, the detection information on all the abnormal shadow candidates having been detected in this manner is sent to the doctor who makes a final decision to determine if the case is truly positive or falsely positive. However, if there are a great number of detections as mentioned above, the entire information has to be examined by the doctor and this is not efficient. For an experienced doctor, it is efficient, in some cases, to examine suspicious images on a priority basis rather than the images clearly indicative of a abnormal shadow.

If an abnormal shadow is present close to an edge of the breast image or the area of the shadow is small, the doctor cannot get a good view of the shadow, which will tend to be overlooked.

The problem to be solved in the present invention is to ensure efficient radiographic interpretation work by sending only the detection information on the abnormal shadow candidate and/or abnormal shadow candidate of lower visibility wherein it is difficult to determine whether the candidates are truly positive or not, thereby preventing the shadow from being overlooked by a doctor.

**MEANS FOR SOLVING THE PROBLEMS**

The invention described in Claim 1 is an abnormal shadow candidate display method including steps of:

detecting abnormal shadow candidates by analyzing an medical image;
extracting the abnormal shadow candidates to be displayed, out of the abnormal shadow candidates having been detected; and
displaying the detection information on the abnormal shadow candidates having been extracted.

The invention described in Claim 2 is the abnormal shadow candidate display method described in Claim 1 wherein, in the aforementioned extraction step,
the characteristic amount of the image of the abnormal shadow candidates having been detected is calculated;
the priority of the abnormal shadow candidates to be displayed is determined according to this characteristic amount of the image; and
out of the detected abnormal shadow candidates, the abnormal shadow candidates of higher priority are extracted on a priority basis.

The invention described in Claim 3 is the abnormal shadow candidate display method described in Claim 2 wherein, in the aforementioned extraction step, the contrast between the abnormal shadow candidates and their surrounding regions is calculated as the characteristic amount of the image, and a higher priority is given to the abnormal shadow candidates of smaller contrast.

The invention described in Claim 4 is the abnormal shadow candidate display method described in Claim 2 wherein, in the aforementioned extraction step, the area of the abnormal shadow candidates region is calculated as the characteristic amount of the image, and a higher priority is given to the abnormal shadow candidates of smaller area.

The invention described in Claim 5 is the abnormal shadow candidate display method described in Claim 2 wherein, in the aforementioned extraction step, the distance from the edge of the aforementioned medical image to the detected position of the abnormal shadow candidate is calculated as the characteristic amount of the image, and a higher priority is given to the abnormal shadow candidates of shorter distance.

The invention described in Claim 6 is the abnormal shadow candidate display method described in any one of Claims 2 through 5, further characterized in that the abnormal shadow candidate display method contains a step of identifying a subject region from the aforementioned medical image and classifying the subject region into a plurality of regions; and in the aforementioned extraction step, priority is determined according to the classification region wherein the abnormal shadow candidate has been detected among the aforementioned subject regions having been identified.

The invention described in Claim 7 is the abnormal shadow candidate display method described in Claim 2 wherein, in the aforementioned extraction step, the characteristic amount of the shape of an abnormal shadow candidate is calculated as the characteristic amount of the image, and a higher priority is given according to the characteristic amount of the shape.

The invention described in Claim 8 is the abnormal shadow candidate display method described in Claim 2 wherein, in the aforementioned extraction step, the density of the abnormal shadow candidate region is calculated as the characteristic amount of the image, and a higher priority is given to the characteristic amount of lower density.

The invention described in Claim 9 is the abnormal shadow candidate display method described in Claim 2 wherein, in the aforementioned extraction step, at least one of the area of the abnormal shadow candidates region, density of the abnormal shadow candidates region, the contrast of the region to the surrounding region, the shape of the abnormal shadow candidate, and the distance from the edge of the aforementioned medical image to the abnormal shadow candidate detected position is calculated as the characteristic amount of the image, and a priority order is given according to the characteristic amount having been calculated.

The invention described in Claim 10 is the abnormal shadow candidate display method described in Claim 1 or 2 wherein, in the display step, the detection information of the abnormal shadow candidate having been extracted is displayed together with the aforementioned medical image.

The invention described in Claim 11 is the abnormal shadow candidate display method described in Claim 10 wherein the medical image is captured by the phase contrast radiographing method.

The invention described in Claim 12 is the abnormal shadow candidate display method described in Claim 10 or 11 wherein in the display step, the life-size medical image obtained by reducing the medical image to the same size as that of the subject is displayed.

The invention described in Claim 13 is the abnormal shadow candidate display method described in Claim 12 wherein a reference image is created from the medical image and the image obtained by superimposing the detection information on the created image is displayed together with the life-size image.

The invention described in Claim 14 is the abnormal shadow candidate display method described in Claim 1 wherein, in the extraction step, the abnormal shadow candidates to be displayed are identified and extracted, out of the abnormal shadow candidates having been detected, based on the threshold value preset to determine if an candidate is to be displayed or not.

The invention described in Claim 15 is the abnormal shadow candidate display method described in Claim 14, wherein the aforementioned threshold value contains a first threshold value and a second threshold value and, in the extraction step, and the abnormal shadow candidates to be displayed are determined based on the first and second threshold value and are extracted out of the abnormal shadow candidates having been detected.

The invention described in Claim 16 is the abnormal shadow candidate display method described in Claim 15, wherein the first threshold value is intended to delete falsely positive candidates, while the second threshold value is intended to delete truly positive candidates, the extraction step further characterized by extracting the abnormal shadow candidates remaining after deletion of the falsely and truly positive candidates from the abnormal shadow candidates having been detected by the first and second threshold values.

The invention described in Claim 17 is the abnormal shadow candidate display method described in Claim 15 or 16, wherein the first or second threshold value can be set for each doctor interpreting the radiograph.

The invention described in Claim 18 is the abnormal shadow candidate display method described in Claim 15 or 16, wherein the first threshold value is set commonly for all the doctors interpreting the radiograph, and the second threshold value can be set for each doctor interpreting the radiograph.

The invention described in Claim 19 is the abnormal shadow candidate display method described in any one of Claims 14 through 18, wherein the threshold value contains a third threshold value for determining the degree of visibility on the image, and the extraction step extracts the abnormal shadow candidates having been determined by the third threshold value as having a lower visibility, out of the detected abnormal shadow candidates.

The invention described in Claim 20 is an medical image processing system including:

an abnormal shadow candidate detecting device for analyzing an medical image and detecting abnormal shadow candidates;

a control device for extracting the abnormal shadow candidates to be displayed, out of the abnormal shadow candidates having been detected by the abnormal shadow candidate detecting device; and

a display device for displaying the detection information on the abnormal shadow candidates having been extracted.

The invention described in Claim 21 is the medical image processing system described in Claim 20 wherein the display device displays the detection information on the extracted abnormal shadow candidates together with the medical image.

The invention described in Claim 22 is the medical image processing system described in Claim 21 wherein the aforementioned medical image is a medical image captured by the phase contrast radiographing method.

The invention described in Claim 22 is the medical image processing system described in Claim 20 wherein the aforementioned control device identifies and extracts the abnormal shadow candidates to be displayed, out of the abnormal shadow candidates having been detected, based on the threshold value preset to determine if an candidate is to be displayed or not.

## EFFECTS OF THE INVENTION

According to the inventions described in Claims 1, 2, 10, 11, and 20 through 22, the amount of display of the detection information (indication of the detection position by a marker or the like) on the display screen can be reduced by decreasing the number of the abnormal shadow candidates to be displayed, so that the detection information that can be easily examined by a doctor is provided. Further, the doctor receives only the detection information on the abnormal shadow candidates wherein it is difficult to determine whether the candidates are truly positive or not, and careful examination by the doctor is required; the abnormal shadow candidates of low visibility or the abnormal shadow candidates falling into both of these categories; or the doctor receives the aforementioned detection information on a priority basis, whereby the efficiency of radiographic interpretation work by the doctor is enhanced, hence diagnostic precision is improved.

According to the invention described in Claim 3, the doctor receives on a priority basis the detection information on abnormal shadow candidates that require careful examination by the doctor to determine whether the candidates are truly positive or not, because of insufficient contrast, whereby the efficiency of radiographic interpretation work by the doctor is enhanced. Further, detection information of insufficient contrast signifies lower visibility. Accordingly, when priority is given to such abnormal shadow candidates, the possibility of an abnormal shadow being overlooked by the doctor can be eliminated.

According to the invention described in Claim 4, the doctor receives on a priority basis the detection information on abnormal shadow candidates that require careful examination by the doctor to determine whether the candidates are truly positive or not, because of insufficient area, whereby the efficiency of radiographic interpretation work by the doctor is enhanced. Further, detection information of insufficient area signifies lower visibility. Accordingly, when priority is given to such abnormal shadow candidates, the possibility of an abnormal shadow being overlooked by the doctor can be eliminated.

According to the invention described in Claim 5, the doctor receives on a priority basis the detection information on abnormal shadow candidates that are so located as to be easily overlooked by the doctor. This arrangement eliminates the possibility of an abnormal shadow being overlooked by the doctor.

According to the invention described in Claim 6, the doctor receives on a priority basis the detection information on abnormal shadow candidates that are located in the region of low visibility. This arrangement eliminates the possibility of an abnormal shadow being overlooked by the doctor.

According to the invention described in Claim 7, the doctor receives on a priority basis the detection information on abnormal shadow candidates which are so shaped that it is difficult to determine whether the candidates are truly positive or not, and careful examination by the doctor is essential. This arrangement enhances the efficiency of radiographic interpretation work by the doctor. Further, visibility is reduced in some cases, depending on the shape. Thus, display of such abnormal shadow candidates on a priority basis eliminates the possibility of an abnormal shadow being overlooked by the doctor.

According to the invention described in Claim 8, the doctor receives on a priority basis the detection information on abnormal shadow candidates of lower density and low visibility. This arrangement eliminates the possibility of an abnormal shadow being overlooked by the doctor.

According to the invention described in Claim 9, the doctor receives on a priority basis the detection information on abnormal shadow candidates wherein it is difficult to determine whether the candidates are truly positive or not, and careful examination by the doctor is required; the abnormal shadow candidates that is difficult for the doctor to visually recognize or the abnormal shadow candidates falling into both of these categories. Further, the priority is determined according to each characteristic amount. This allows priority to be determined in a comprehensive manner, with consideration given to various forms of decision factors.

According to the invention described in Claims 12 and 13, a life-size image suited for doctor's diagnosis is provided for the purpose of radiographic interpretation by the doctor. A reference image different from the life-size image is also provided as a reference for abnormal shadow candidate detection information. Thus, the image for easy radiographic interpretation (diagnosis) by doctor is provided in the manner for easy interpretation.

According to the invention described in Claims 14 through 19, the number of the abnormal shadow candidates to be displayed can be adjusted in conformity to the skill of the doctor interpreting a radiograph or the style of radiographic interpretation such as the order of radiographic interpretation. Further, these related specifications can be customized.

**BRIEF DESCRIPTION OF THE DRAWINGS**

Fig. 1 is a drawing representing the structure of the medical image processing system in the present embodiment.
Fig. 2 is a diagram describing the phase contrast radiographing method.
Fig. 3 is a diagram describing an image obtained by the phase contrast radiographing method.
Fig. 4 is a diagram representing the structure of the image processing apparatus.
Fig. 5 is a diagram showing the regions for classification of breast images.
Fig. 6 is a flow chart representing a process of determining the candidates to be displayed, wherein this process is implemented by the image processing apparatus.
Fig. 7 is a flow chart representing a process of detecting the abnormal shadow candidates, wherein this process is implemented by the image processing apparatus.
Fig. 8 is a diagram describing the method of calculating the characteristic amount representing the degree of complexity at the margin.
Fig. 9 is a drawing showing an example of displaying the result of detecting the abnormal shadow candidates.
Fig. 10 is a flow chart representing a process of determining the candidates to be displayed, wherein this process is implemented by the image processing apparatus.
Fig. 11 is a drawing showing an example of displaying the abnormal shadow candidate detection information.
Fig. 12 is a drawing showing another example of displaying the abnormal shadow candidate detection information.
Fig. 13 is a flow chart representing the flow of the process of detecting the abnormal shadow candidates (one process pattern),
Fig. 14 is a diagram representing the detection algorithm using a filter bank.
Fig. 15 is a flow chart representing the flow of the process of detecting the abnormal shadow candidates using a filter bank.
Fig. 16 is a flow chart representing the flow of the process of detecting the abnormal shadow candidates using a characteristic amount.

Fig. 17 is a flow chart representing the flow of the process of detecting the abnormal shadow candidates (another process pattern), wherein this process is implemented by the image processing apparatus.

Fig. 18 is a diagram showing an example of displaying the abnormal shadow candidate detection information by each processing pattern.

Fig. 19 is a flow chart representing the flow of the process of detecting the abnormal shadow candidates.

Fig. 20 is a diagram showing an example of displaying the abnormal shadow candidate detection information through the process of detection in conformity to the lesion variant to be detected.

## DESCRIPTION OF REFERENCE NUMERALS

100 Medical image processing system
1 Image generating apparatus
2 image processing apparatus
21 Control section
26 Image processing section
27 Abnormal shadow candidate detecting section
3 Printer
4 Image server
4a Image DB
5 Viewer

## BEST MODE FOR CARRYING OUT THE INVENTION

The following describes the present embodiment with reference to an example wherein a breast image formed by capturing the image of breasts is used as an medical image.

The structure will be described in the first place.

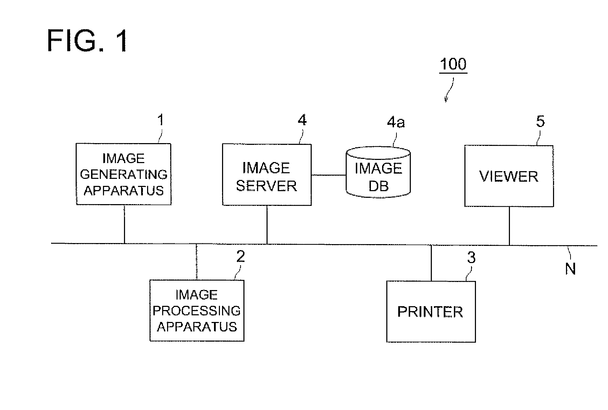

Fig. 1 is a drawing representing the structure of the medical image processing system 100.

The medical image processing system 100 captures the medical image of a subject, detects abnormal shadow candidates by applying image processing to the medical image, and supplies a doctor with the detection information together with the medical image.

As shown in Fig. 1, the medical image processing system 100 includes an image generating apparatus 1, an image processing apparatus 2, a printer 3, an image server 4, and a viewer 5. These devices 1 through 5 are connected in such a way as to exchange information among them via the communication network N configured in an medical institution such as LAN ⟨Local Area Network⟩. The communication network N conforms to the DICOM (Digital Imaging and Communication in Medicine) standard.

The following describes the aforementioned component devices 1 through 5:

The image generating apparatus 1 captures the image of the subject and generates the digital data of the captured image (medical image). For example, the modality of the CR (Computed Radiography), FPD (Flat Panel Detector), CT, MRI, reading apparatus designed specifically for a cassette, and film digitizer can be applied. In this embodiment, a CR system including the phase contrast radiographing apparatus designed specifically for radiographing both breasts is used as the image generating apparatus 1, whereby the data on phase contrast breast image is created.

The image generating apparatus 1 conforms to the DICOM standard. Various forms of information (e.g. patient information on the patient for who the medical image has been captured, radiographing information on radiographing and examination, examination information) to be attached to the generated medical image can be inputted from the outside, and can be automatically generated. The image generating apparatus 1 attaches the aforementioned accompanying information as header information to the generated medical image, which is then sent to the image processing apparatus 2 via the communication network N. If the image generating apparatus 1 does not conform to the DICOM code, a DICOM conversion apparatus (not illustrated) can be used to input the accompanying information into the image generating apparatus 1.

The following describes the phase contrast radiographing method and apparatus:

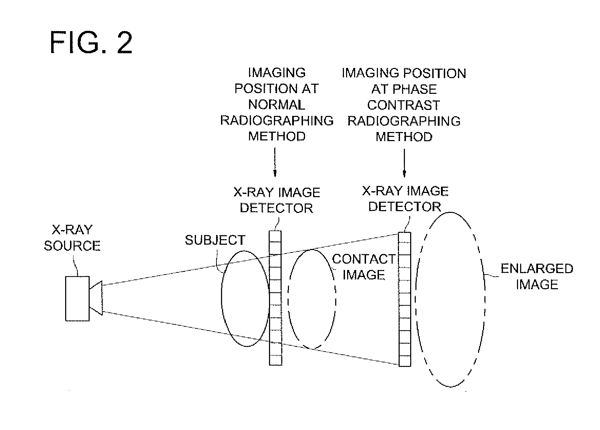

As shown in Fig. 2, the phase contrast radiographing method pertains to macrophotography wherein enlarged image is captured by setting a distance between the subject and detector (e.g. a cassette or FPD (Flat Panel Detector)). When this method is used, the image having been obtained is an enlarged version of the life-size image of the subject as shown in Fig. 2. This arrangement provides an image of high resolution. Assume, for example, that magnification is used wherein the image captured is magnified two times that in the conventional radiographing method wherein an image is captured with the subject placed in contact with the detector. This arrangement provides the same resolution as that of the image being read using the pixel of half the size, even if the image is read using the same pixel size as that of the conventional radiographing method.

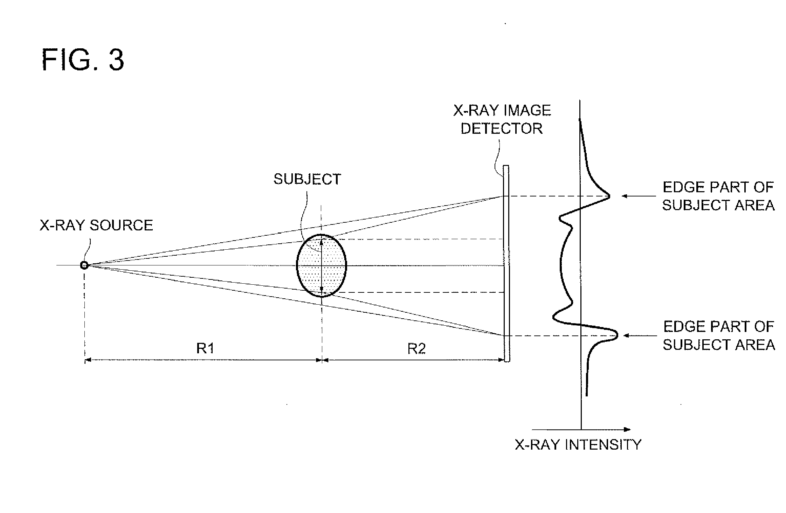

As shown in Fig. 3, when the distance from the X-ray source to the subject is R1, and the distance from the subject to the detector is R2, the rate of magnification is expressed by (R1 + R2)/R1. Since information on this magnification rate is used for subsequent processing, it is included in the accompanying information of the breast image.

As shown in Fig. 3, a slight refraction occurs when the X-ray passes through the subject. Edge enhancement is produced by this refraction on the boundary portion of the image region of the subject (phase contrast effect). Thus, the enlarged image obtained from the edge of the lesion is an image of high visibility wherein the boundary portion of the lesion edge is edge-enhanced.

The image processing apparatus 2 applies various forms of image processing to the medical image supplied from the image generating apparatus 1, and analyzes the medical image, whereby abnormal shadow candidates are detected.

Based on the medical image data received from the image processing apparatus 2 or image server 4, the printer 3 outputs the medical image to the recording medium such as a film.

The image server 4 has an image DB4a. The medical image (original image) generated by the image generating apparatus 1 and medical image (processed image) processed and received from the image processing apparatus 2 are stored in the image DB4a, whereby the input and output thereof is managed.

The viewer 5 is a display device used by a doctor for diagnosis, and includes an LCD (Liquid Crystal Display). The viewer 5 acquires the instructed medical image from the image server 4 in response to the operation instruction by the doctor, and displays the image. Alternatively, the result of the abnormal shadow candidate having been detected by the image processing apparatus 2 is received and displayed.

The following describes the details of the image processing apparatus 2 of the present invention.

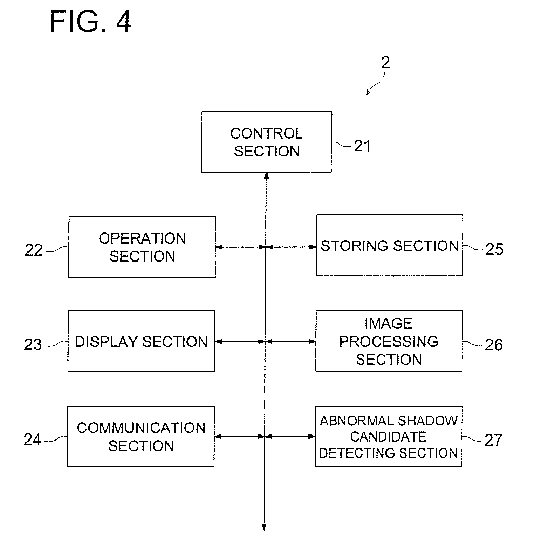

Fig. 4 is a diagram representing the internal structure of the image processing apparatus 2.

The image processing apparatus 2 includes a control section 21, operation section 22, display section 23, communication section 24, storing section 25, image processing section 26 and abnormal shadow candidate detecting section 27.

The control section 21 includes a CPU (Central Processing Unit) and RAM (Random Access Memory). Various forms of control programs are read from the storing section 25 by the CPU and are loaded on the RAM. Integrated control of the execution of the processing is performed according to the program, and operations of various sections are controlled in a concentrated manner. For example, the decision on the candidates to be displayed (to be described later) is processed according to the program of processing the decision on the candidates to be displayed related to the present invention. The control device can be implemented by the co-operation between the control section 21 and the program of processing the decision on the candidates to be displayed.

The operation section 22 includes a keyboard and mouse. It generates the operation signal corresponding to the key operation and mouse operation and outputs it to the control section 21.

The display section 23 contains an LCD and others. Various types of display such as the operation screen at the time of image processing and the medical image subsequent to the processing are displayed in response to the instruction of the control section 21.

The communication section 24 is provided with the communication interface such as a router and modem, and performs communication with the external apparatus on the communication network N according to the instruction of the control section 21. For example, it receives the medical image to be processed from the image generating apparatus 1, and sends the processed medical image to the image server 4 or printer 3.

The storing section 25 stores various forms of control programs, parameters required for execution of the program and the data on the result of processing.

The image processing section 26 applies the processes of graduation conversion and sharpness adjustment to the medical image according to the image processing program. In the case of breasts, the image processing section 26 further applies the process of alignment for composing the images of both breasts in adjacent positions. Alignment can be carried out according to the conventionally known technique, wherein, for example, the position of each breast image is shifted in the vertical direction so that the shape of the pectoral muscle extracted from the breast image will exhibit bilaterally symmetrically.

The image processing section 26 creates the display image so as to display the abnormal shadow candidate detection information superimposed on the breast image. The breast image obtained by the phase contrast radiographing method is an enlarged version of the life size of the subject. The enlarged version can be displayed directly. However, the check of actual size of lesion also plays a crucial role for examination at the time of diagnosis. Thus, the image is often displayed after having been reduced to the actual size (life size). The breast image is accompanied by the information on the magnification rate through the image generating apparatus 1 at the time of radiographing. When the life size of the subject is used, that is, the life-size image of the subject is displayed, the process of reduction is applied according to the information on magnification rate, whereby a life-size image is created.

When the life-size image is generated, it is preferred to select the output sampling pitch in such a way that the reading sampling pitch of the phase contrast enlarged image (called "A" tentatively) and the output sampling pitch (called "B", including the beam diameter and display pixel size at the time of film writing) will meet the relationship of the magnification rate = A/B, because this selection eliminates the need of carrying out the process of interpolating the read image data. If the phase contrast enlarged radiographing has been performed at a magnification rate of 1.75 and reading has been performed at 43.75 $\mu$m, the output sampling pitch is preferably 25 $\mu$m. If the output sampling pitch meeting this relationship cannot be used, the process of reduction interpolation corresponding to the output sampling pitch is applied, whereby a life-size image is generated.

The image processing section 26 creates the display image which is formed by superimposing on the breast image the abnormal shadow candidate detection information detected by the abnormal shadow candidate detecting section 27. To put it more specifically, based on the detection information inputted from the abnormal shadow candidate detecting section 27, the image processing section 26 identifies the abnormal shadow candidate detection position of the created life-size image and superimposes on that position the marker image (an arrow or box) indicating the abnormal shadow candidate detection information.

The abnormal shadow candidate detecting section 27 analyzes the medical image, extracts the subject region, classifies it into a plurality of regions, calculates the characteristic amount of the image from the medical image, and detects the abnormal shadow candidate based on this characteristic amount of the image.

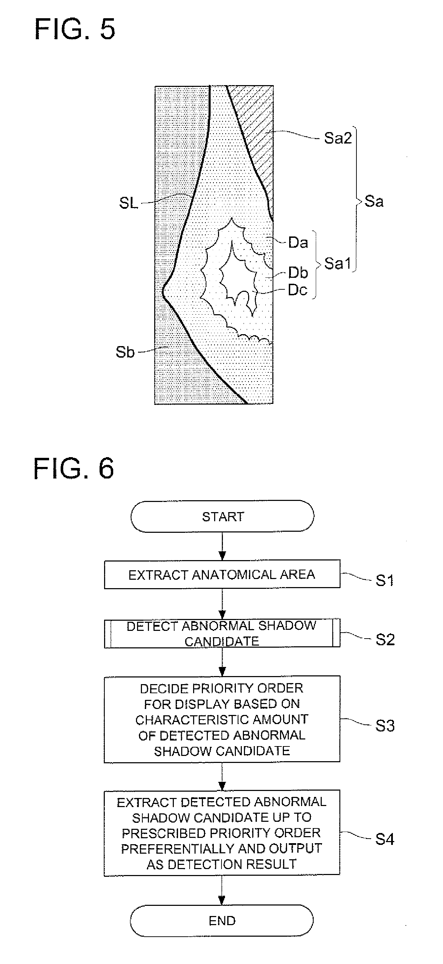

The following describes the procedure of extracting each region from the image data with reference to Fig. 5.

Fig. 5 shows a breast image S captured in the oblique position (hereinafter referred to as "MLO"). The subject region Sa is extracted from the breast image S, and the breast region Sa1 and pectoral muscle region Sa2 are extracted from the subject region Sa. After that, the breast region Sa1 is classified into three regions Da, Db and Dc.

(1) The abnormal shadow candidate detecting section 27 obtains the variance histogram of the pixel value in the breast image S and determines the threshold value using the discriminant analysis method (a method of determining the threshold value to ensure that the discrimination ratio (variance ratio) of the intra-class variance and inter-class variance will be maximized in the two classes, when the variance histogram is classified into two classes). The breast image S is binarized using the threshold value having been determined.

In this case, the blank region (region directly exposed to the X-ray without corresponding to the subject portion) exhibits a high degree of density in the breast image S, and the value is turned onto "l" by binarization. In other areas, the value is expected to be turned onto "0" by binarization. Thus, the breast image S can be classified into the subject region Sa and other blank regions Sb by binarization. It should be noted that, when radiographing is performed in the front direction (hereinafter referred to as "CC"), the pectoral muscle image is not captured into the subject portion, and therefore, the subject region Sa means the breast region Sa1. Further, the boundary between two regions (subject region and blank region) due to binarization is identified as a skin line SL.

(2) When the direction of radiographing is MLO, the pectoral muscle image is captured into the subject portion, and therefore, the pectoral muscle region Sa2 is identified from the subject region Sa. For the pectoral muscle region Sa2, the density inclination in the subject region Sa is calculated, for example. From this density inclination, the subject region Sa is classified into the pectoral muscle region Sa2 and breast region Sa1. As shown in the Unexamined Japanese Patent Application Publication No. 2001-238868, it is also possible to make such arrangements that a local region is set, and a threshold value is set based on the pixel value in the local region so that the subject region Sa is binarized, whereby the pectoral muscle region Sa2 and breast region Sa1 are identified.

(3) The breast region Sa1 is classified into three regions Da, Db and Dc.

The breast region contains a mammary gland and fat, and the density varies according to the density thereof. When diagnosis is made by a doctor, the breast region is classified according to the density. It is often classified into the tree categories as high-density region including a great deal of fat wherein the percentage of the mammary gland contained therein is less than 10%, mid-density region wherein the percentage of the mammary gland contained therein is 10% or more and less than 50%, and low-density region wherein the percentage of the mammary gland contained therein is 50% or more. On the image, the abnormal shadow appears white as low density. Accordingly, if there is any lesion region in the low-density area corresponding to the whitest portion of the greater pectoral muscle or in the mid-density region that appears whitish, visual observation of a abnormal shadow is difficult.

Thus, with reference to the density of the pectoral muscle region Sa2, the breast region Sa1 is classified into a high-density region Da, a mid-density region Db and a low-density region Dc. To put it more specifically, a variance histogram of the pectoral muscle region Sa is created and the region of comparatively uniform density is extracted from its shape. Based on this average density as a threshold value, the region is classified into regions Da, Db and Dc. For example, the region remaining after deletion of the region of higher density than the threshold value is assumed as Dc, Of the regions of high density having been deleted, the high-density region having higher density than the threshold value by 30% or more of the threshold value is assumed as Db, and the high-density region having higher density than the threshold value by 60% or more of the threshold value is considered as Da, whereby the region can be divided into regions Da, Db and Dc.

The following describes the method of detecting the abnormal shadow candidates.

The abnormal shadow candidate detecting section 27 performs the process of detection according to the detection program of the algorithm conforming to the type of the abnormal shadow to be detected. In the case of a breast image, the abnormal shadow candidate detecting section 27 detects the shadow candidate of the tumor or cluster of micro calcification as the cancerized portion of a breast cancer.

A conventionally known algorithm can be used as the abnormal shadow candidate detection algorithm. For example, a method using an iris filter or a Laplacian filter disclosed in the Unexamined Japanese Patent Application Publication No. H10-91758 (Collected Research Papers of the Institute of Electronics, Information and Communication Engineers (D-II), Vol. J76-D-II, NO. 2 pp. 241 - 249, 1993) can be utilized as the abnormal shadow candidate detection algorithm for a breast image. Further, the methods using a morphology filter (Collected Research Papers of the Institute of Electronics, Information and Communication Engineers (D-II), Vol. J71-D-II, NO. 7 pp. 1170 - 1176, 1992), a Laplacian filter (Collected Research Papers of the Institute of Electronics, Information and Communication Engineers (D-II), Vol. J71-D-II, NO. 10 pp. 1994 - 2001, 1998), and a triple ring filter can be utilized as an algorithm for detecting the shadow candidate of micro calcification cluster, for example.

A shadow candidate detection method of micro calcification cluster in the breast image will be described an example of the method of detecting the abnormal shadow candidate.

On the breast image, the micro calcification cluster shadow appears as a shadow wherein low-density micro calcification parts having changing density in substantially conical shape are collected (clustered). Based on this density characteristics, the regular square regions are set sequentially with respect to the medical image, and a triple ring filter having a specific spectral pattern is applied to each of these regions (also called "region of interest") as a micro calcification cluster detection filter, whereby primary detection of the abnormal shadow candidate is carried out. The size of this region of interest can be set according to the lesion type to be detected.

The triple ring filter contains three ring filters wherein the intensity component and direction component of the density inclination are determined in advance when the change in density exhibits an ideal conical form. In the first place, around the pixel of interest, a representative value for the intensity component and direction component of the density inclination is obtained from the pixel value in each region of each ring filter. Primary detection of an approximately conical image region of changing density is performed, based on the difference between the representative values and the intensity component and direction component of the density inclination determined in advance for each ring filter.

When a candidate region has been specified in the primary detection, various forms of characteristic amount of the image such as contrast, standard deviation, average density value, curvature, fractal dimension, circularity degree, and area in this candidate region are calculated. Based on the characteristic amount, a decision step is taken to determine whether or not it is a truly positive abnormal shadow (truly lesional region, whereas the shadow of a normal tissue which may be misinterpreted as a lesion is called a falsely positive shadow). A multivariate analysis method can be used for this step of decision. For example, the characteristic amount of the image calculated from the shadow which is known to be truly positive in advance is used as learning data, and multivariate analysis is configured. Various forms of characteristic amount of the image calculated from the shadow candidate to be determined are inputted into this multivariable analysis, thereby getting an indicator value showing the possibility of being a truly positive candidate. This indicator value is compared with the threshold value prepared to determining a truly positive candidate, whereby a step is taken to determine whether the candidate is truly positive or not. The region containing the candidates having been determined to be truly positive is outputted as the candidate region for the micro calcification cluster shadow.

[EMBODIMENT 1]

The first embodiment for the operation of the medical image processing system 100 will be described below:

In the first place, the following describes the flow from the generation to the storage of the medical image.

In the first place, an image is captured in the image generating apparatus 1. A medical image (an example of the breast image will be taken as an example for the sake of explanation) is generated. As the information related to the breast image having been generated, the breast image is accompanied by detailed information including the patient information such as the name, age and sex, radiographing information such as information on the tube voltage and breast compression rate at the time of radiographing, inspection information such as the information on inspection date and time, and breast image generation information including the information on image reading conditions.

The breast image containing the aforementioned accompanying information is outputted from the image generating apparatus 1 to the image processing apparatus 2.

Image processing required for the breast image is carried out in the image processing apparatus 2. In the meantime, the step for determining the candidates to be displayed (to be described later) is applied to the breast image.

The abnormal shadow candidates are detected and the candidates to be displayed are extracted from the abnormal shadow candidate having been detected.

Referring to Fig. 6, the following describes the process wherein the image processing apparatus 2 determines the candidates to be displayed:

In the process of determining the candidates to be displayed as shown in Fig. 6, the subject region contained in the medical image, and the pectoral muscle region and breast region in the subject region are outputted in the abnormal shadow candidate detecting section 27 (Step S1).

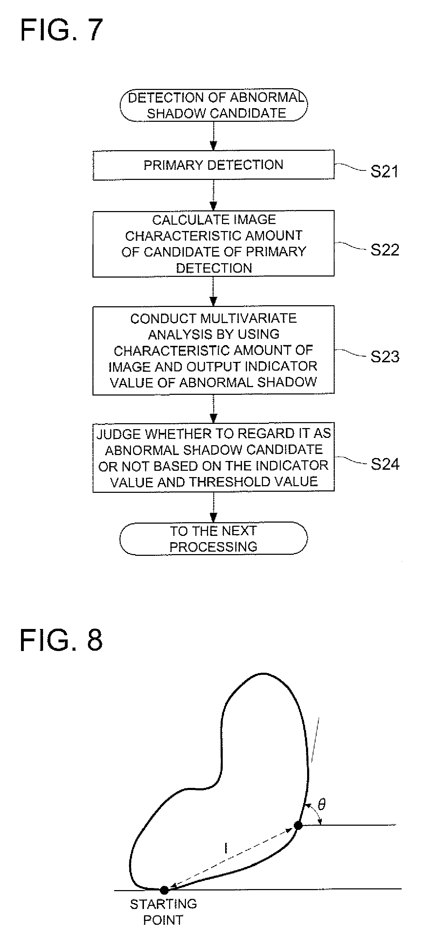

In the abnormal shadow candidate detecting section 27, the process of abnormal shadow candidate detection is applied to the breast region having been extracted (Step S2). The process of abnormal shadow candidate detection will be described with reference to the flow chart of Fig. 7. In the first place, the regions of interest as the unit for detection processing are sequentially set with respect to the breast region. Primary detection is carried out inside these regions of interest (Step S21). The method of the primary detection has already been described, and will not be repeated to avoid duplication.

The characteristic amount of the image is calculated with respect to the region for the abnormal shadow candidate having been subjected to primary detection. What are calculated as the characteristic amounts of the image include at least the area of the abnormal shadow candidate region, standard deviation, average density, curvature, fractal dimensions, circularity degree, contrast to the surrounding region, complexity of the margin, the classification region wherein the abnormal shadow candidate is detected in the primary detection (corresponding to any one of the regions Da through Dc in Fig. 5) (Step 522).

Contrast can be obtained from the difference in density between the abnormal shadow candidate region and its surrounding region (difference in pixel value). The area can be gained from the number of pixels in the abnormal shadow candidate region, while the distance from the image edge can be calculated from the distance (the number of pixels) from the image edge on the chest wall side to the abnormal shadow candidate detection position.

Circularity degree is calculated from the following equation (1). Circularity degree C indicates that, as the value is closer to 1, the abnormal shadow candidate is closer to a circle, in other words, closer to the lesion part (more truly positive). When the value is smaller compared to 1, the graphic representation is complicated and a careful examination is required to determine whether the candidate is truly positive or not.

$$\text{Circularity degree } C = 4\pi \times Ar/L^2 \ \dots \ (1)$$

wherein Ar is an area (number of pixels constituting the abnormal shadow candidate region), and L is a circumferential length (length of the circumference formed by pixels constituting the outline of the abnormal shadow candidate region)

Further, the complexity of the margin denotes the expansion coefficients $a_k$ and $b_k$ calculated by the following equation (2) when the periodic function showing the contour of the abnormal shadow candidate as shown in Fig. 8 is subjected to Fourier expansion. As the expansion coefficients $a_k$ and $b_k$ are greater, the margin of the abnormal shadow candidate region is more distorted.

[Mathematical Formula 1]

$$\theta_n(l) = \sum_k \{a_k\cos(2\pi kl/L) + b_k\sin(2\pi kl/L)\} \ \cdots(2)$$

$a_k$ and $b_k$: complexity of the margin
L: circumferential length (length of the circumference formed by pixels constituting the outline of the abnormal shadow candidate region)
$\Theta_n(l)$: what is obtained by Fourier expansion of the normalization function of the periodic function having a period of L

This is followed by the step of inputting the characteristic amount to the multivariable analysis configured to output the indicator value representing the possibility of the characteristic amount of the image being an abnormal shadow in advance, whereby the indicator value showing the possibility of the truly positive abnormal shadow is obtained (Step S23). Then, based on the indicator value having been obtained and the threshold value, a step is taken to determine whether or not an abnormal shadow candidate is present Step S24). After that, the system proceeds to the Step S3 of Fig. 6.

In the Step S3 of Fig. 6, based on the characteristic amount of the image calculated with respect to the abnormal shadow candidates in the control section 21, the priority of the abnormal shadow candidates to be displayed, out of these abnormal shadow candidates, is determined (Step S3). Here the abnormal shadow candidates to be displayed are the candidates except for those which have been determined as being clearly truly positive or falsely positive. They are the candidates wherein it is difficult to determine if they are truly or falsely positive, those which are difficult to observe, or those which tend to be overlooked by a doctor.

For example, when the contrast and area are small, it is difficult to determine if the candidate is truly positive or not. The final decision by the doctor interpreting a radiograph is essential in many cases. Further, the visibility of this region cannot be said to be satisfactory, and the shadow tends to be overlooked by the doctor. In some of the abnormal shadows which are determined to be truly positive in the final phase, the boundary of the margin is unclear (less sharp), a thin streak appears on the margin or a distorted figure called a spicula appears. In this case, there is an increase in the complexity of the margin. If the complexity of the margin is small, it is difficult to determine whether the shadow is abnormal or not. Further, in the case of a tumor, as the circularity degree is greater, the possibility of being a tumor is higher. If the circularity degree is smaller, it is more difficult to determine if the shadow denotes a tumor (true positive) or not. Even in the same breast region Sa1, the low-density region Dc is generally of smaller density. Accordingly, the abnormal shadow of lower density appearing on the screen is more difficult to identify when it is present in the low-density region Dc than when it is present in the high-density region Da. This applies to the case of the density of the abnormal shadow candidate itself. That is, as the density is lower, visibility is lower. Thus, the candidate tends to be overlooked by the doctor. Further, the abnormal shadow present in the breast region close to the edge of the image is more apt to be overlooked by the doctor because of its particular position.

Thus, the factors for determining the order of priority include contrast, area, average density, the classification region of the abnormal shadow candidate position, distance from the image edge and complexity of the margin. Accordingly, the order of priority is determined in a comprehensive manner with consideration given to these factors.

To put it more specifically, the learning data including the characteristic amount of the shadow to be given a higher priority and the characteristic amount of the shadow to be given a lower priority is prepared in advance. A multivariable analysis is configured, as exemplified by a neural network that outputs 0 through 1 (lower priority as the indicator value is closer to 0, and higher priority as the indicator value is closer to 1) as an indicator value of the priority. The learning data is prepared in such a way that a higher order of priority is assigned as the contrast, area, the average density of the candidate region, circularity degree, the distance from the edge of the image or the complexity of the margin is smaller. Hence the multivariable analysis is adjusted accordingly. If the breast region Sa1, identification is more difficult in the order of Dc, Db and Da. Thus, the multivariable analysis is configured so that the indicator value has a higher priority in this order.

The characteristic amount of abnormal shadow candidates for which the order of priority is to be determined is inputted into the aforementioned multivariable analysis. The indicator value outputted from the multivariable analysis and the threshold value corresponding thereto are compared. The abnormal shadow candidates having exceeded the threshold value, that is, the candidates in the higher order of priority than a predetermined level are determined to be an abnormal shadow candidate to be displayed.

When the priority order has been determined, the abnormal shadow candidates detected in the process of detection in Step S2 are extracted in the order of priority (Step S4), and are outputted as the final result of detection.

The result of detection is sent from the image processing apparatus 2 to the image server 4 where it is stored. The breast image (having been processed for radiographic interpretation) is subjected to data processing for display such as alignment, reduction and superimposition by the image processing section 26, whereby an image for display is generated. The generated image for display is sent from the image processing apparatus 2 to the viewer 5, wherein the image is displayed. In addition to being displayed on the viewer 5, the image can be outputted to the film by the printer 3. In other words, "display" contains the concept of outputting the image on the film and displaying the same.

The breast image by the phase contrast radiographing method is radiographed in an enlarged version, and therefore, the image size is greater than that of the normal radiographing. It can be displayed as it is. However, assume that the life-size image obtained by reducing it to the same size as the life size of the subject is created and is displayed on the viewer 5. At the time of data processing for display, the information on the magnification rate at the time of enlarged radiographing is read out of the accompanying information of the breast image. Based on this magnification rate, the rate of reducing the breast image is determined in the image processing section 26. For example, when the magnification rate is two times, the reduction rate is determined to be 1/2 times. In this case, the area of the reduced image is one fourth that of the original image. A marker image showing the position of detection as the abnormal shadow candidate detection information is superimposed on this life-size image, whereby an image for display is created. This is displayed on the viewer 5.

A readout pixel size is added to the accompanying information. It is also possible to make such arrangements that, based on this readout pixel size, the optimum display site can be selected based on the pixel size of the viewer. For example, when the accompanying information contains the information of magnification rate "2" and readout pixel size "50 $\mu$m", the suitable display site of the life-size image is found in an apparatus having a pixel size of 1/2 x 50 $\mu$m = 25 $\mu$m. This is because the pixel for readout corresponds to the display pixel at a ratio of 1 to 1, without the need of applying the process of reduction interpolation, and hence the image deterioration during interpolation does not occur. If the image processing section 26 obtains the pixel size of each viewer on the network in advance, a suitable display site can be selected easily.

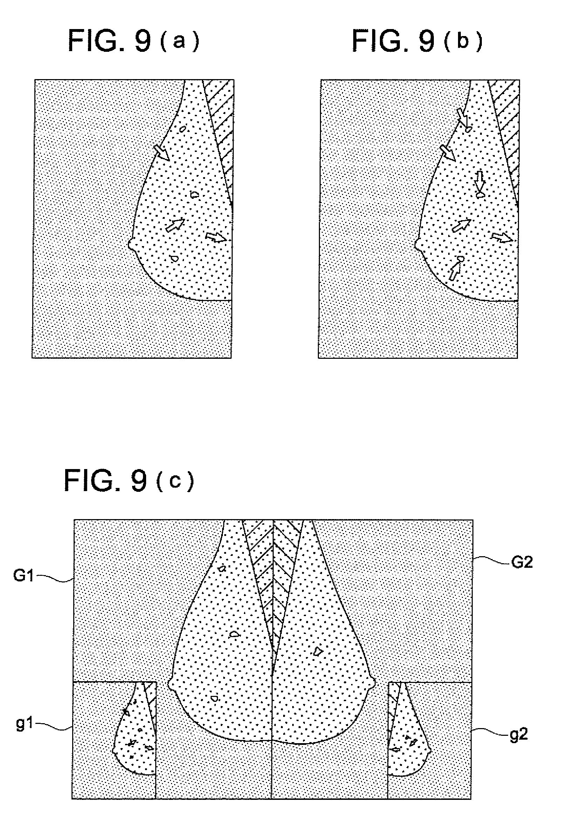

Fig. 9 shows an example of display.

Fig. 9 (a) shows an example of displaying the result of detecting the abnormal shadow candidates whose priority order exceeds a predetermined level. Fig. 9 (b) shows an example of displaying the result of detecting all the abnormal shadow candidates. Reduction in the number of display items on the screen and easy observation are ensured by displaying only the abnormal shadow candidates whose priority order exceeds a predetermined level, as compared with displaying all the abnormal shadow candidates having been detected, as will be apparent from Figs. 9 (a) and (b).

Figs. 9 (a) and (b) give an example wherein the result of detecting the abnormal shadow candidate is displayed directly on the medical image by the marker information (arrow in the diagram) indicating the position of the abnormal shadow candidate having been detected. It is also possible to make such arrangements that the reduced sub-images (indicated by g1 and g2 in the diagram) is produced and is displayed in such a way that it does not overlap with the subject region of the main images (indicated by G1 and G2 in the diagram), as shown in Fig. 9 (c). To be more specific, the reference image is formed of the abnormal shadow candidate detection information which is superimposed on the screen wherein the life-size image is further reduced. In this case, the region showing the result of detection is smaller than that of Fig. 9 (a). According to the present invention, the number of the abnormal shadow candidates to be displayed can be reduced, and therefore, this is especially efficient. Fig. 9 (c) shows that the images for both breasts have been aligned so that the image ends on the chest wall are adjacent to each other.

As described above, in the present embodiment, the clearly truly positive or falsely positive abnormal shadow candidates are removed from the abnormal shadow candidates having been detected, and detection information is displayed only for the candidates wherein it is difficult to determine whether they are truly or falsely positive and the decision should be left to the final decision by the doctor, and the candidates of low visibility which tend to be overlooked by a doctor. This arrangement reduces the number of abnormal shadow candidates to be displayed, and hence provides a display for easy observation, whereby the efficiency of radiographic interpretation by the doctor is enhanced.

Thus, the doctor is allowed to check the clearly truly positive abnormal shadows by visual inspection. When detecting the abnormal shadows of low visibility that cannot be easily observed, the doctor is permitted to refer to the result of detecting the abnormal shadow candidates provided by the medical image processing system 100. When all the abnormal shadow candidates having been detected are to be displayed, the doctor is required to examine all the candidates including the clearly truly positive ones. This is a heavy burden on the doctor. To avoid this, only the result of detecting the abnormal shadow candidates that cannot be easily detected is supplied, as described above. This arrangement prevents candidates from being overlooked by the doctor and enhances the efficiency of radiographic interpretation by the doctor.

Especially the margin of the lesion in the image obtained by the phase contrast radiographing method is known to ensure a substantial improvement of the visibility over the conventional breast image. This arrangement is preferably used because it provides a substantial reduction in the possibility of overlooking a clear legion (truly positive candidate region) free from annotations such as arrows or boxes.

It is also possible to make such arrangements that only the breast image having been generated is supplied to the doctor, who performs radiographic interpretation (diagnosis) of this image, and records the result of radiographic interpretation. After that, the information on the abnormal shadow candidates having been detected is provided. The doctor compares it with the result of previous radiographic interpretation (diagnosis) and can correct the result. The display can be controlled to permit this procedure.

The aforementioned embodiment is an example wherein the present invention is preferably applied, without the present invention being restricted thereto, however.

In the above example, for example, the priority order is determined by the multivariable analysis method in a comprehensive manner. Without the present invention being restricted thereto, the priority order is determined for each decision factor of each abnormal shadow candidate, for example, by giving a priority order to the contrast and circularity degree in ascending order. The numbers of the priority orders for each decision factor are added together. The candidates having a smaller additional value can be extracted on a priority basis. In this case, addition can be made by assigning weights to the decision factors of greater importance, whereby the priority order can be optimized.

In the aforementioned description, the abnormal shadow candidate detecting device and control device are realized in the image processing apparatus 2 and the display device is realized in the viewer 5. Without being restricted thereto, each device can be implemented by any of the components of the medical image processing system 100.

Especially when radiographing is performed by the image generating apparatus 1 according to the phase contrast method disclosed in the Unexamined Japanese Patent Application Publications Nos. 2001-238871, 2001-311701, 2002-85389 and 2001-299733, the phase contrast effect is known to bring about a substantial improvement of visibility of the margin of the lesion in the image having been obtained, as compared to the conventional breast image. This arrangement is preferably used because it ensures a substantial reduction in the possibility of overlooking the clear lesion (truly positive candidate region) free of annotation.

In the aforementioned description, the "region of low density and low visibility" refers to the region wherein there is a small amount of light reaching the eyes of the doctor engaged in radiographic interpretation during the interpretation. Thus, in the case of radiographic interpretation by a film and viewing light box, it refers to the film region wherein there is a small amount of transmitted light. It corresponds to the black portion on the film, whereas the blank portion on the film belongs to the "region of high density and high visibility". On the other hand, in the case of radiographic interpretation by a viewer, the "region of low density and low visibility" refers to the region of low density (low drive level).

Generally, the region wherein there is a small amount of the X-ray passing through the subject at the time of radiographic interpretation corresponds to the blank portion and is reproduced and displayed at a high degree of luminance. As the region of low visibility for the doctor interpreting a radiograph, the region wherein a greater amount of X-ray has passed through the subject is required to be extracted out of the image data having been radiographed.

[Embodiment 2]

The second embodiment for the operation of the medical image processing system 100 will be described below:

In the first place, the following describes the flow from the generation to the storage of the medical image.

As described with reference to the first embodiment, an image is captured in the image generating apparatus 1. A medical image (an example of the breast image will be taken as an example for the sake of explanation) is generated. As the information related to the breast image having been generated, the breast image is accompanied by detailed information including the patient information such as the name, age and sex, radiographing information such as information on the tube voltage and breast compression rate at the time of radiographing, inspection information such as the information on inspection date and time, and breast image generation information including the information on image reading conditions.

The breast image containing the aforementioned accompanying information is outputted from the image generating apparatus 1 to the image processing apparatus 2.

Image processing required for the breast image is carried out in the image processing apparatus 2. In the meantime, the step for determining the candidates to be displayed is applied to the breast image. The abnormal shadow candidates are detected and the candidates to be displayed are extracted from the abnormal shadow candidate having been detected. Here, the abnormal shadow candidate to be displayed are ones wherein it is not easy to determine whether they are truly positive or falsely positive, or candidates that must be left to the decision of the doctor in the final phase, or candidates that are of low visibility and cannot be easily observed by the doctor, or being located where they cannot be easily found out.

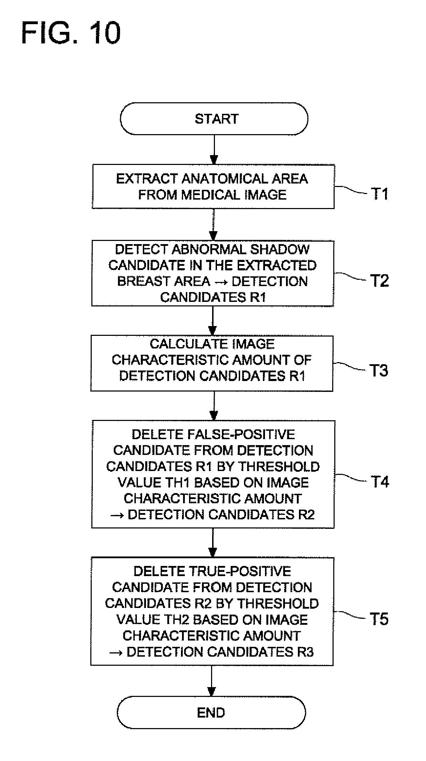

Referring to Fig. 10, the following describes the process wherein the image processing apparatus 2 determines the candidates to be displayed:

In the process of determining the candidates to be displayed as shown in Fig. 10, the breast region, pectoral muscle region and others are extracted from the medical image by the abnormal shadow candidate detecting section 27 (Step T1). The abnormal shadow candidates are detected in the extracted breast region (Step T2). In this case, the abnormal shadow candidates having been detected is assumed as detected candidates R1. The abnormal shadow candidates can be detected, for example, by the method of detecting the micro calcification cluster shadow candidates in the breast image, as described with reference to the first embodiment.

This is followed by the step of calculating the image characteristic amount of the detected candidates R1 (step T3). The image characteristic amount including at least the standard deviation of the pixel value in the candidate region, average density, curvature, fractal dimension, circularity degree, area, contrast with the surrounding region, complexity of the margin, distance from the image edge, and the classification region (present in any of the regions Da, Db or Dc of Fig. 5) wherein the abnormal shadow candidates have been detected is calculated. The image characteristic amount is used as a decision factor to determine the abnormal shadow candidates wherein it is difficult to determine whether they are truly positive or not, or the candidates of low visibility.

Contrast is obtained from the difference in density (difference in pixel values) between the abnormal shadow candidate region and its surrounding region, the area is obtained from the number of pixels in the abnormal shadow candidate region, and the distance from the image edge is obtained from the distance (number of pixels) from the image edge on the chest wall to the abnormal shadow candidate detection position.

Circularity degree is calculated from the formula (1) describe with reference to the first embodiment. Complexity corresponds to the expansion coefficients ak and bk calculated from the formula (2) described with reference to the first embodiment, when the periodic function representing the contour of the abnormal shadow candidate is subjected to Fourier expansion, as shown in Fig. 8.

In the control section 21, the falsely positive candidates (candidates having a higher possibility of being falsely positive) in the detected candidates R1 is detected, based on the calculated image characteristic amount, using the first threshold value TH1 which is preset to detect the falsely positive candidates and is stored in the storing section 25. This is followed by the step of deleting these falsely positive candidates from the detected candidates R1 (Step T4). The candidates remaining after deletion of these falsely positive candidates from the candidates are assumed as detected candidates R2.

To put it more specifically, the indicator value representing the possibility of being truly positive is calculated by multivariable analysis, based on various image characteristic amounts such as contrast, standard deviation, average density, curvature, area, fractal dimension, circularity degree, and complexity of the margin calculated in the candidate region. For example, a decision logic is configured via the neural network wherein learning data is provided based on the image characteristic amount calculated from the shadow which is clearly truly positive, or the image characteristic amount which is calculated from the shadow of low visibility on the image and which is determined to be likely to be overlooked by the doctor. Each image characteristic amount calculated from the shadow candidates to be determined is input into this decision logic, thereby getting an indicator value representing the possibility of the shadow candidates being truly positive. The falsely positive candidates are deleted in response to the result of comparison between this indicator value and threshold value TH1.

The following describes the conditions wherein, assuming that threshold value TH1 = 0.25, for example, the candidates having an indicator lower than this level are determined as falsely positive ones. When the indicator value (referred to as "S") for the truly positive property to be outputted at the time of detection is outputted as a value 0 through 1 (lower possibility of being truly positive as the value is closer to 0, and higher possibility of being truly positive as the value is closer to 1), the detected candidates R1 wherein the indicator value S is smaller than TH1 (= 0.25) are detected as falsely positive candidates.

The falsely positive candidates having been detected are deleted from the detected candidates R1, and the remaining detected candidates R2 are detected. Then the control section 21 detects the truly positive candidates (referring to the candidates having a higher possibility of being truly positive) from the detected candidates R2, based on the image characteristic amount of the detected candidates R2, using the second threshold value TH2 which is preset to delete the truly positive candidates and is stored in the storing section 25. These truly positive candidates are deleted from the detected candidates R2 (Step T5). The detected candidates remaining after deletion of the truly positive candidates from these detected candidates R2 are assumed as R3.

Consider, for example, that there are the conditions wherein, assuming that threshold value TH2 = 0.85, the candidates having an indicator value higher than this level are determined as truly positive ones. In the aforementioned example, the detected candidates R3 wherein indicator value S is greater than TH2 (= 0.85) are detected as truly positive candidates from the detected candidates R2.

To be more specific, the detected candidates R3 are the candidates obtained by removing the falsely positive candidates and truly positive candidates out of the detected candidates R1 having been detected by the process of detection, in other words, the detected candidates R3 are the candidates wherein it is difficult to determine whether the candidates are truly positive or not, and careful examination by the doctor is required.

The threshold values TH1 and TH2 used to delete the falsely or truly positive candidates can be set for each radiographic interpretation. To put it more specifically, the setting information of threshold values TH1 and TH2 can be inputted through the operation section 22. The setting information of threshold values TH1 and TH2 is associated with the ID specific to each doctor interpreting a radiograph and is stored in the storing section 25. When the ID of the doctor interpreting a radiograph is inputted at the time of radiographic interpretation, the threshold values TH1 and TH2 associated with the doctor interpreting a radiograph are read by the control section 21 and are used for the aforementioned processing.

For example, if a doctor wishes to minimize the number of the falsely positive candidates to be displayed, the threshold value TH1 or TH2 is set in response to the skill and style of the doctor interpreting a radiograph; for example, the setting of the threshold value TH1 is changed from 0.25 to 0.3. This arrangement makes it possible to adjust the number of the abnormal shadow candidates to be displayed. Thus, the abnormal shadow candidates can be displayed in the manner best suited to the skill of the doctor interpreting a radiograph.

The result of detecting the detected candidates R3 having been extracted by the threshold values TH1 and TH2 is sent from the image processing apparatus 2 to the image server 4, and is stored. In the meantime, the medical image and the result of detection are sent from the image processing apparatus 2 to the viewer 5 and are displayed on the viewer 5.

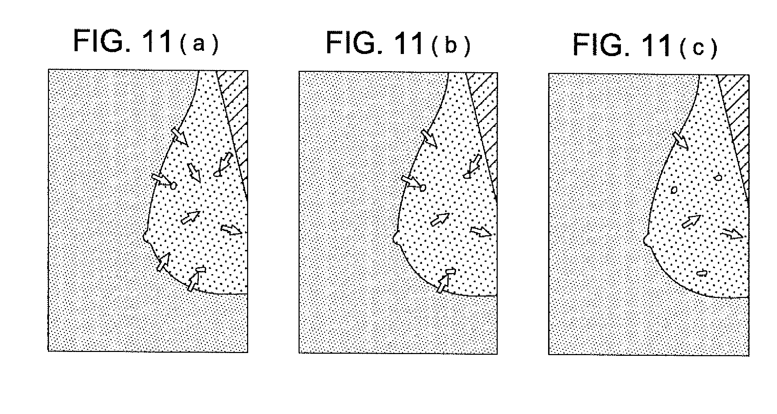

Fig. 11 shows an example of display.

Fig. 11 (a) is an example of displaying the detected candidates R1. Fig. 11 (b) is an example of displaying the detected candidates R2. Fig. 11 (c) is an example of displaying the detected candidates R3. As can be seem from Figs. 11 (a) through (c), falsely positive candidates are deleted from the detected candidates R1 (Fig. 11 (b))- As the truly positive candidates are deleted (Fig. 11 (c)), the number of markers indicating the detecting position of the candidates is reduced on the display screen for medical image, whereby an easy-to-see display is obtained. Further, efficient radiographic interpretation is ensured because the abnormal shadow candidates which are clearly falsely positive and truly positive candidates are removed from the candidates to be displayed.

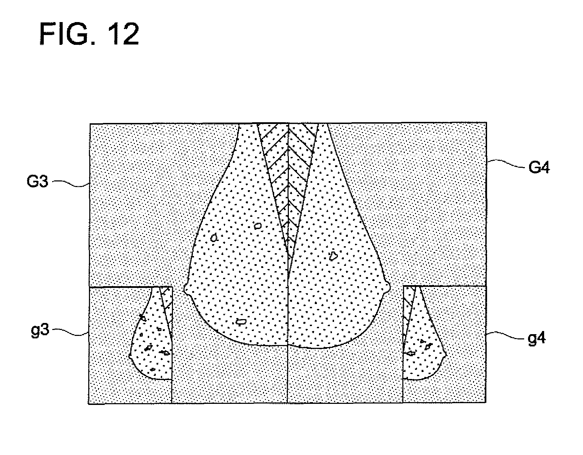

Fig. 11 (c) shows an example wherein the abnormal shadow candidate detection information is displayed on the breast image. It is also possible to make arrangements as follows. As shown in Fig. 12, the breast image without detection result being shown is used as the main images (images G3 and G4 in the diagram). Detection information is superimposed on the sub-images obtained by reducing the size thereof (images g3 and g4), and the resulting images are displayed so that they will not overlap with the subject region of the main images. In this case, the detection information display region is smaller than that in the form of display in Fig. 11 (c). However, according to the present invention, the number of the abnormal shadow candidates to be displayed can be reduced, and therefore, this arrangement can be especially used effectively. Fig. 12 shows an aligned image subjected to positioning processing wherein the image of both breasts are aligned so that chest wall side are adjacent to each other.

In the aforementioned embodiment, to extract abnormal shadow candidates wherein it is difficult to determine whether they are truly or falsely positive and the final decision should be left to the doctor, explanation has been made using an example of calculating the indicator value representing the possibility of being truly positive, based on the image characteristic amount of the abnormal shadow candidates. To extract the abnormal shadow candidates of lower visibility on the screen because of still lower density or location at still lower visibility, the indicator value representing the degree of visibility is calculated. In this case as well, decision logic by the multivariable analysis is configured wherein the image characteristic amount of the abnormal shadow candidates of lower visibility is used as learning data. Thus, calculation is made to get the indicator value that represents the degree of visibility, based on the image characteristic amount of the abnormal shadow candidates to be determined. A third threshold value TH3 is created to determine if the visibility is low or not, and the abnormal shadow candidates of low visibility are only required to be determined and extracted by the threshold value TH3 in the phase of extracting any one of the detected candidates R1 through R3 in the abnormal shadow candidates. This arrangement makes it possible to provide only the detection information of the candidates wherein it is difficult to determine whether they are truly positive or not and the final decision should be left to the doctor, and/or the candidates of low visibility which tend to be overlooked by a doctor.

As described above, according to the present embodiment, the clearly truly or falsely positive candidates are deleted from the abnormal shadow candidates detected by the process of detection. Detection information is displayed only on the candidates wherein it is difficult to determine whether they are truly or falsely positive and the final decision should be left to the doctor, and the candidates of low visibility which tend to be overlooked by a doctor. This arrangement reduces the number of the abnormal shadow candidates to be displayed. Thus, easy-to-see display is provided and efficiency in radiographic interpretation is enhanced.

Use of two threshold values allows abnormal shadow candidates to be classified into three categories; clearly truly positive ones, doubtful whether truly positive or falsely positive and clearly falsely positive ones. The order of radiographic interpretation can be determined according to the style of radiographic interpretation (diagnosis) by the doctor or the physical condition of the doctor. For example, it is possible to adjust the schedule in such a way that only the clearly truly positive ones and clearly falsely positive ones are subjected to radiographic interpretation. After the physical conditions of the doctor have been recovered on the following date, radiographic interpretation will be applied to the candidates wherein it is difficult to determine whether they are truly positive or not and the careful decision of the doctor is required.

Thus, clearly truly positive candidates are subjected by visual observation of the doctor, and the candidates of low visibility which cannot be found easily can be detected by referring to the result of detecting the abnormal shadow candidates provided by the medical image processing system 100. When all the abnormal shadow candidates having been detected are to be displayed, the doctor have to check all the abnormal shadow candidates including the ones that can be clearly determined as truly positive ones by visual observation. This is a heavy burden on the doctor. To avoid this, as described above, the information on the result of detecting only the abnormal shadow candidates that cannot be easily found out is provided. This will prevent the candidates from being overlooked by the doctor and will improve the doctor's radiographic interpretation.

When the phase contrast radiographing method is used to capture the image, the margin of the lesion portion in the image is known to exhibit a substantial enhancement of visibility through the effect of phase contrast, as compared to the case of the conventional breast image. This can be preferably used since it provides a substantial reduction in the possibility of overlooking the clear lesion portion (truly positive candidate region) having no indication of detection information.

The aforementioned embodiment shows an example to which the present invention is preferably applied without the present invention being restricted thereto.

For example, in the above description, the abnormal shadow candidate detecting device and control device are implemented through the image processing apparatus 2, and the display device is implemented through the viewer 5. Without being restricted thereto, each device can be implemented by any of the components of the medical image processing system 100.

In the aforementioned embodiment, both the threshold values TE1 and TH2 can be set in response to the requirements of the doctor interpreting a radiograph. It is also possible to arrange such a configuration that the threshold value TH1 for detecting the falsely positive candidates is set as a common value for all the doctors interpreting a radiograph, while the threshold value TH2 for detecting the truly positive candidates can be set according to the requirements of each doctor. For example, the threshold value TH1 is set to the value preset by a manufacturer or the value standardized in a hospital so that the results of detection can be normalized. The threshold value TH2 is configured in such a way that the results of detection can be adjusted to suit the skill of each doctor. This will provide specifications best suited to the environment of interpreting.

The threshold value TH1 and the like are assumed as the threshold values for the indicator value outputted as a result of decision logic in the multivariable analysis. Without being restricted thereto, a threshold value TH1 can be created for each image characteristic amount. For each the image characteristic amount, the abnormal shadow candidates extracted by the threshold value can be the object for display.

Further, in the present embodiment, it is also possible to arrange such a configuration that only the breast image having been generated is sent to the doctor at first, radiographic interpretation (diagnosis) is performed based on this image, and the result is recorded by the doctor. After that, display and control can be carried out so that the information on the abnormal shadow candidates having been detected is provided, and is compared with the result of radiographic interpretation by the doctor and the result can be corrected.

In the first radiographic interpretation, the doctor identifies the presence of the region wherein it is difficult to determine whether they are truly positive or not, in addition to the truly positive region. When displaying the result of detecting the abnormal shadow candidates, the threshold value corresponding to this region is inputted, and the result of detection is displayed, whereby the distinction between truly and falsely positive candidates in the abnormal shadow candidates can be made by referring to the result of detection. As compared to the method of displaying all the results of detection, more reliable diagnosis can be achieved quickly in a shorter time. Further, the level of the doctor's skill can be improved by using this invention.

Abnormal shadow candidates can be determined in a shorter time by changing the threshold value in response to the improvement (level up) in the doctor's skill of interpreting the radiograph (diagnosis).

[Other embodiments of the present invention]

The following describes the operation of the aforementioned medical image processing system 100 with reference to other embodiments of the present invention. In the present embodiment, when the abnormal shadow candidates are detected through different detection processes by a plurality of detection algorithms, the abnormal shadow candidates to be displayed are extracted from the abnormal shadow candidates having been detected in response to the detection process. The detection information of the extracted abnormal shadow candidates is displayed and outputted. The following describes an example of this procedure:

In the first place, the flow from generation to storage of the medical image will be described:

As discussed with reference to the first and second embodiments, radiographing is performed in the image generating apparatus 1 so that a medical image (an example of breast image will be described here) is generated. As the information related to the breast image having been generated, the breast image is accompanied by detailed information including the patient information such as the name, age and sex, radiographing information such as information on the tube voltage and breast compression rate at the time of radiographing, inspection information such as the information on inspection date and time, and breast image generation information including the information on image reading conditions.

The breast image containing the aforementioned accompanying information is outputted from the image generating apparatus 1 to the image processing apparatus 2.

In the image processing apparatus 2, the breast image having been inputted through the communication section 24 is stored in the storing section 25 and image processing required for this breast image is applied by the image rocessing section 25. In the meantime, a process of detecting the abnormal shadow candidates is applied to this breast image by the abnormal shadow candidate detecting section 27.

Detection of the abnormal shadow candidates is carried out by a combination of two detection algorithms. Three processing patterns are available by a combination of two detection algorithms whereby the detection processes are made different. A desired processing pattern can be set by the technician or doctor interpreting a radiograph.

The first through third processing patterns are available. In the first one, after detection by one detection algorithm, another detection algorithm is used to apply a process of detection to the image regions except for the region that has been determined by the detection to be falsely positive. The second pattern is the same as the first one except that the order of processing by the detection algorithm is reversed. In the third pattern, detection by the first algorithm and other detection algorithms is carried out in parallel. If any one of the algorithms has detected, this result is counted as the results of detection.

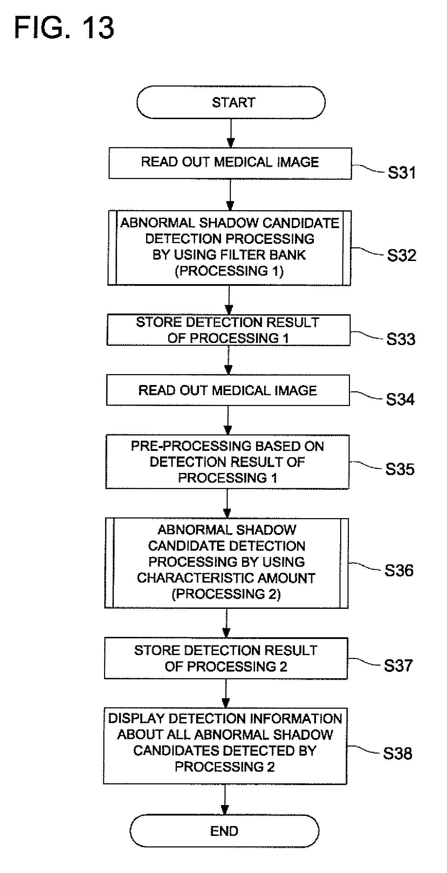

Referring to Fig. 13, the following describes the first pattern. After that, other processing patterns (second and third processing patterns) will be described.

Fig. 13 is a flow chart representing the flow of the process of detecting the abnormal shadow candidates in the first process pattern.

As shown in Fig. 13, the breast image stored in the storing section 24 is read out in the abnormal shadow candidate detecting section 27 (Step S31). The abnormal shadow candidate detection processing (hereinafter referred to as "processing 1") is applied to this breast image (Step S32), wherein a filter bank is used as the detection algorithm.

The following describes the detection algorithm of the filter bank wherein the circular or linear pattern is to be detected: (See Collected Research Papers of the Institute of Electronics, Information and Communication Engineers D-II, Vol. J87-D-II, No. 1 pp. 186 - 196).

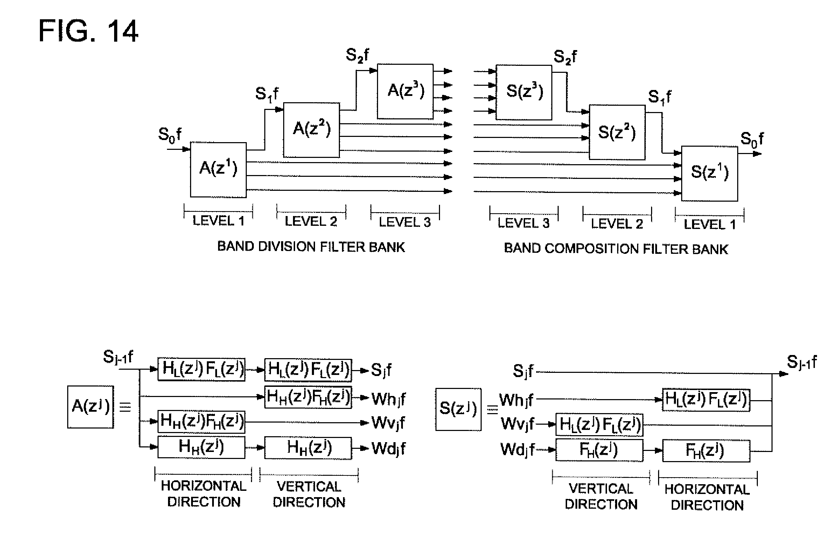

Fig. 14 shows the filter bank incorporating the concept of multiple resolution in wavelet analysis. The original image is $S_0f$, and the level of this resolution is zero. $H_L(z^j)$, $F_L(z^j)$ is a low-path filter on the resolution level and $H_H(z^j)$ $F_H(z^j)$ is high-path filter. Further, $S_jf$, $Wh_jf$, $Wv_jf$ and $wd_jf$ denote the image of smoothed portion, the image of horizontal portion, the image of vertical portion and the image of diagonal portion on resolution level j, respectively. Division of the low-frequency component is repeated on the band division side of the filter bank, whereas the components are synthesized on the band composition side of the filter bank. In this filter bank, each filter is represented by the mathematical expressions (3) through (6).

[Mathematical Formula 2]

$$H_L\left(z^j\right)F_L\left(z^j\right)=\frac{1}{4}\left(z^{2j}+2+z^{-2j}\right) \qquad (3)$$

[Mathematical Formula 3]

$$H_H\left(z^j\right)F_H\left(z^j\right)=\frac{1}{4}\left(-z^{2j}+2-z^{-2j}\right) \qquad (4)$$

[Mathematical Formula 4]

$$H_H\left(z^j\right)=\frac{1}{2}\left(z^j-z^{-j}\right) \qquad (5)$$

[Mathematical Formula 5]

$$F_H\left(z^j\right)=\frac{1}{2}\left(-z^j+z^{-j}\right) \qquad (6)$$

Here $H_H(z)$ denotes a first-order difference filter, and $H_H(z)F_H(z)$ indicates a second-order difference filter. Thus, the images of the horizontal and vertical portions on the resolution level j correspond to the images formed by adding the second-order difference filter in the vertical direction to the image of the smoothening portion on the resolution level j-1, and adding the second-order difference filter in the horizontal direction thereto respectively. The image on the diagonal portion corresponds to the image formed by adding the first order difference filter in the horizontal and vertical directions. To be more specific, the images on these portions correspond to the elements of the Hessian matrix represented by the following formula (7).

[Mathematical Formula 6]

$$H = \begin{bmatrix} \dfrac{\partial^2 f}{\partial x^2} & \dfrac{\partial^2 f}{\partial x \partial y} \\ \dfrac{\partial^2 f}{\partial x \partial y} & \dfrac{\partial^2 f}{\partial y^2} \end{bmatrix} \approx \begin{bmatrix} Wv_j f & Wd_j f \\ Wd_j f & Wh_j f \end{bmatrix} \qquad (7)$$

The minimum eigenvalue of the Hessian matrix wherein elements are made up of the images of the horizontal, vertical and diagonal portions of the resolution level j is used as the circular pattern enhancement image $\lambda\,min_j\,(x, y)$ of the resolution level j (Formula 8).

[Mathematical Formula 7]

$$\lambda\,min_j(x, y) = \text{the minimum eigenvalue of } \begin{bmatrix} Wv_j\,f & Wd_j\,f \\ Wd_j\,f & Wh_j\,f \end{bmatrix} \quad (8)$$

Further, the maximum eigenvalue of the Hessian matrix is used as the circular/linear pattern enhancement image $\lambda\,max_j\,(x, y)$ of the resolution level j (Formula 9).

[Mathematical Formula 8]

$$\lambda\,max_j(x, y) = \text{the maximum eigenvalue of } \begin{bmatrix} Wv_j\,f & Wd_j\,f \\ Wd_j\,f & Wh_j\,f \end{bmatrix} \quad (9)$$

Use of the circular pattern enhancement image $\lambda\,min_j\,(x, y)$ and the circular/linear pattern enhancement image $\lambda\,max_j\,(x, y)$ allows the circular pattern and the circular/linear pattern of difference sizes to be detected.

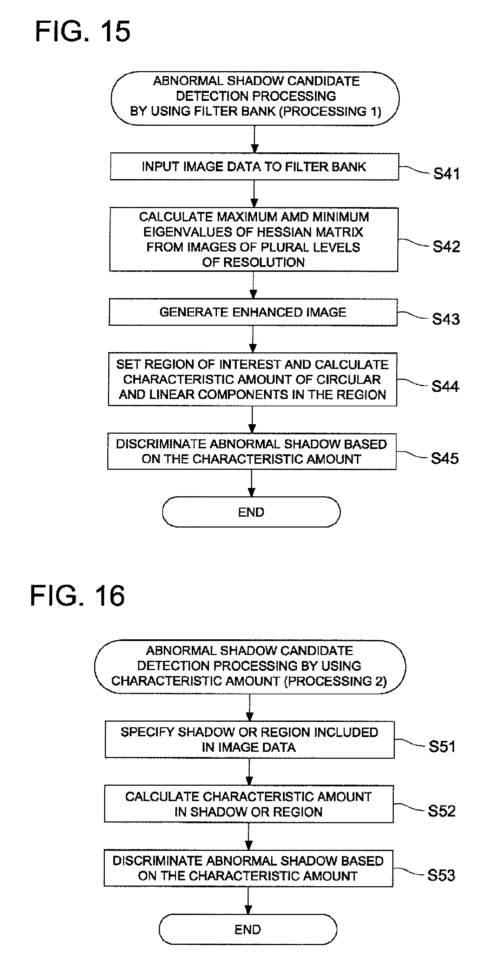

Referring to Fig. 15, the following describes the process of detecting the abnormal shadow candidates by the detection algorithm of the filter bank described above:

In the first place, the medical image to be detected is inputted into the filter bank (Step S41).

This is followed by the step of calculating the maximum and minimum eigenvalues of the Hessian matrix from the images of a plurality of resolutions obtained from the filter bank (Step S42). The circular/linear patterns of difference sizes are created (Step S43), and the circular and linear components are extracted.

The region of interest (e.g. 5 mm x 5 mm) is set on the enhancement image. The image characteristic amount of the circular/linear components located in the range are calculated (Step S44). The size and distribution of each component are included in the image characteristic amounts of the circular/linear components.

Based on the image characteristic amounts of the circular/linear components, a step is taken to determine if the circular/linear components are truly positive or falsely positive (Step S45).

This decision can be made by decision analysis such as Mahalanobis distance or the like. According to this method, learning data is prepared in advance, and this data provides a clear distinction between the truly and falsely positive candidates. Mahalanobis distance is obtained based on the image characteristic amount of the learning data. Then a decision is made on whether the test item is closer to the truly positive one or falsely positive one. When decision has been made, the system goes to the Step S33 of Fig. 13.

In Step S33, as the result of detecting the abnormal shadow candidates in processing 1, the position and area of the shadow candidates having been determined as truly positive or falsely positive candidates are calculated and are stored in the storing section 25.

The same image as the medical image detected in processing 1 is read from the storing section 25 (Step S34). The information on the result of detecting the abnormal shadow candidates which has been detected in processing 1 is read out from the storing section 25. Based on the result of detection having been read out (position information), preprocessing is carried out to generate the image (called the preprocessed image) formed by removing from the medical image the image region which has been determined as falsely positive in the processing 1 (Step S35). To put it more specifically, in this processing, a flag indicating removal from the objects to be detected is set to each pixel of the image region which has been determined as falsely positive, for example.

The preprocessed image having been generated is subjected to processing (hereinafter referred to as "processing 2") wherein abnormal shadow candidates are detected using the image characteristic amount (Step S36) . The processing 2 uses a detection algorithm whereby the image characteristic amount of the shadow region contained in the image is calculated, and based on the image characteristic amount having been calculated, a step is taken to determine whether the shadow is truly positive or falsely positive. Thus, abnormal shadow candidates are detected.

The following describes the flow of the detection using the detection algorithm of processing 2, with reference to Fig. 16:

In the first place, the abnormal shadow candidate region is specified from the preprocessed image (Step S51). This is done by means of the iris filter disclosed in the Unexamined Japanese Patent Application Publication No. H10-91758, a Laplacian filter (See Collected Research Papers of the Institute of Electronics, Information and Communication Engineers D-II, Vol. J76-D-II, NO. 2, pp. 241 - 249, 1993), a morphology filter (See Collected Research Papers of the Institute of Electronics, Information and Communication Engineers D-II, Vol. J71-D-II, NO. 7 pp. 1170 - 1176, 1992), a Laplacian filter (See Collected Research Papers of the Institute of Electronics, Information and Communication Engineers D-II, Vol. J71-D-II, NO. 10, pp. 1994 - 2001, 1998), or a triple ring filter. Various forms of algorism have been developed to conform to the type of the lesion. Any of these algorithms can be used. It should be noted that a filter is not applicable to the region having been deleted after being identified as the falsely positive one by the preprocessing, and the candidate region is not specified.

When the candidate region has been specified, the image characteristic amount of the specified candidate region is calculated (Step S52). The image characteristic amount includes the area of the candidate region, circularity degree, irregularity, average pixel value, standard deviation, candidate region and its contrast.

The area is represented by the number of pixels constituting the specified candidate region.

The circularity degree is an image characteristic amount representing the complexity of the shape. It is expressed by the following formula (10) wherein S is the area of the candidate region and L is the length (circumference) of the contour of the candidate region.

$$e = 4\pi S/L^2 \quad \dots \quad (10)$$

Alternatively, it can be expressed by the formula (11) wherein U represents the area (overlapping area) formed by overlapping between the circle having the same area as the area S of the candidate region wherein the center of gravity of the candidate region is used as a center, and the candidate region.

$$e = U/S \quad \dots \quad (11)$$

wherein circularity degree "e" is greater as one goes close to the circle and the value is closer to 1.

The irregularity f is expressed by the following formula (12) wherein the circumference of the candidate region is L and the circumference after the circumference of the candidate region has been smoothed is L'.

$$f = L'/L \quad \dots \quad (12)$$

Based on each calculated image characteristic amount, a step is taken to determine whether the abnormal shadow candidate having been detected in processing 2 is truly positive or falsely positive (Step S53). In this decision step, learning data is prepared in advance, and this data provides the image characteristic amount of the candidate images belonging to two groups of truly positive and falsely positive. The Mahalanobis distance from the center of each group to the data to be determined (image characteristic amount having been calculated) is obtained, and a step is taken to determine whether it is closer to the truly positive group or to the falsely positive group. When the result of decision has been obtained, the system proceeds to the Step S37 of Fig. 13.

In Step S37, as the result of detection in processing 2, the information on the position and area of the abnormal shadow candidates is stored in the storing section 25.

Then the control section 21 reads from the storing section 25 the information on the extraction condition set for the processing 1, together with the information on the result of detection in the processing 1. It should be noted that the information on the extraction condition set on the processing 1 is extraction of all the abnormal shadow candidates. To be more specific, based on information of the result of detection having been read out, all the abnormal shadow candidates having been detected are extracted as the candidates to be displayed. The detection information is displayed on the display section 23 (Step S38). The detection information refers to the information on the detection of abnormal shadow candidates to be sent to the doctor. It is exemplified by the marker image indicating the position of detection and text information indicating the items related to abnormal shadow candidates such as the type of the lesion to be displayed, as well as the area.

The above description refers to the flow of processing in the processing 1. The processing 2 is the same as the processing 1 except that the order of processing 1 and processing 2 is reversed. Thus, the detailed description of the second processing pattern will be omitted. The abnormal shadow candidate abstraction condition in the second processing pattern is the same as that in the first processing pattern. To be more specific, processing 1 is applied to the preprocessed image wherein the falsely positive region is deleted in the processing 2. The information is displayed for all the abnormal shadow candidates which have been identified as truly positive in the processing 1.

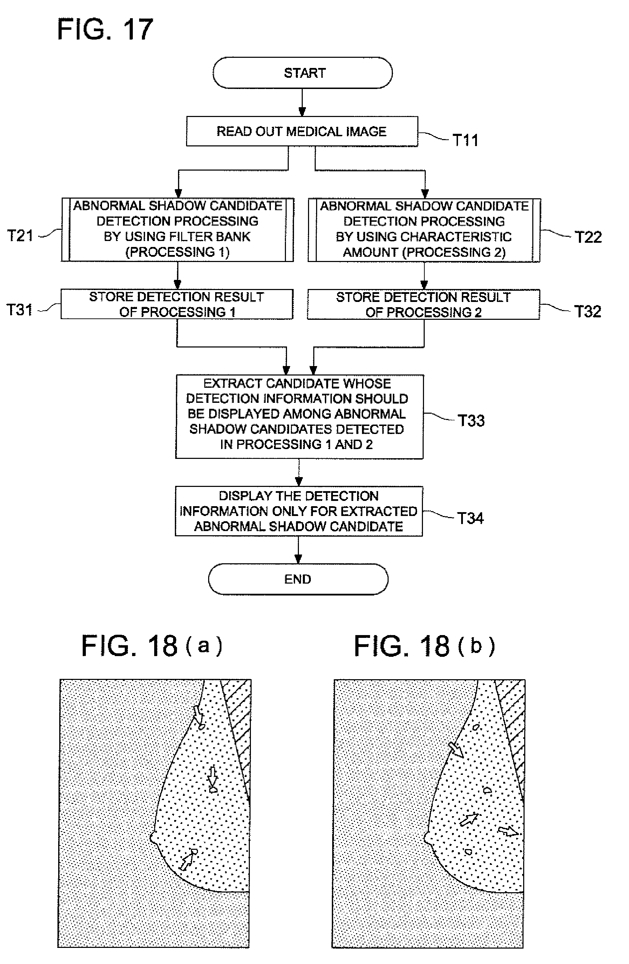

Referring to Fig. 17, the following describes the abnormal shadow candidate detection processing related to the third processing pattern.

As shown in Fig. 17, in the abnormal shadow candidate detecting section 27, the breast image stored in the communication section 24 is read out (Step T11). Then processing of detection by the processing 1 and processing 2 is applied to this breast image independently and in parallel to each other (Steps T21 and 22). The results of detection in the processing of detection are each stored in the storing section 25 (Steps T31 and T32). The details of the processing 1 and 2 have already been described, and will not be described here.

The control section 21 reads the result of detection in processing 1 and 2 from the storing section 25. Of the abnormal shadow candidates having been detected, those candidates whose detection information is to be displayed and outputted are extracted (Step T33).

In the case of the first and second processing patterns, the region determined as being truly positive by one detection algorithm is again detected by another detection algorithm. The region having been determined as being truly positive again by this new detection algorithm is outputted as the final result of detection. To be more specific, the detection is performed in two steps, and this arrangement is expected to increase the possibility that the abnormal shadow candidates contained in the result of detection are truly positive.

In the meantime, in the case of the third processing pattern, one detection algorithm and another algorithm are applied separately and in parallel to each other. The abnormal shadow candidates having been detected by any one of the algorithms are outputted as the final result of detection. Thus, the detection precision is considered to be lower than that of the first or second processing pattern, and there seems to be a higher possibility of the falsely positive candidates being contained in the result of detection. If information is to be displayed for all the abnormal shadow candidates detected in the similar manner as in the first processing pattern, based on such a result of detection, there will be an increased in the number of display items on the display section 23, with the result that the display is not easy to observe.

Thus, in the third processing pattern, the candidates to be displayed are extracted out of the abnormal shadow candidates having been detected, thereby reducing the number of display items of the detection information.

The abnormal shadow candidates for which the detection information is to be displayed are the candidates wherein it is difficult to determine whether they are truly or falsely positive, and the final decision should be left to the doctor. The extraction conditions for extracting such candidates are set up and are stored in the storing section 25. The truly positive shadows which can be clearly identified as such can be found out by the doctor, even if there is no display of the detection information by the image processing apparatus 2. Thus, some doctor wishes to have the detection information on the shadows wherein it is difficult to determine whether they are truly positive or not, not the detection information on the aforementioned shadows. Accordingly, extraction conditions are set up to meet such requirements.

Since it is necessary to provide information on the candidates of low visibility at the detection position or on the image which cannot be easily observed by a doctor, it is also possible to arrange such a configuration that such candidates are extracted.

Image characteristic amounts are used to extract the abnormal shadow candidates to be displayed.

For example, if the contrast and area are smaller, it is difficult to determine whether the candidates are truly positive or falsely positive, and the final decision by the doctor is often essential. In this case, the visibility is also poor and such shadows are often overlooked by the doctor. Further, in the truly positive abnormal shadow, the boundary portion of the marginal section is unclear (less sharp), and fine streaks appear on the marginal section. Alternatively, the distorted shape called "spicula" appears in some cases. This results in an increase in the degree of irregularities on the margin. Conversely, when the irregularities of the margin are smaller, the distinction between truly positive and falsely positive candidates is difficult. In the case of tumor, as the circularity degree is greater, the shadow has a higher possibility of being a tumor. If the circularity degree is smaller, it is more difficult to determine if the shadow indicates a tumor (truly positive shadow) or not.

For the reasons discussed, these image characteristic amounts are used as decision factors to determine the abnormal shadow candidates wherein it is difficult to determine whether the candidates are truly positive or not, and the abnormal shadow candidates of low visibility.

To put it more specifically, the indicator value showing the possibility of being truly positive is calculated by multivariable analysis, based on the image characteristic amounts such as the contrast, standard deviation, average density value, curvature, area, fractal dimension, circularity degree and irregularities of the margin having been calculated in the candidate region. For example, the image characteristic amount calculated from the shadow which has been known as being truly positive in advance is used as learning data, and a decision logic by multivariable analysis such as the neural network is configured. The image characteristic amounts calculated from the shadow candidates to be determined are inputted in this decision logic, thereby getting the indicator value showing the possibility that the shadow candidates are truly positive.

The truly positive and falsely positive candidates are determined in the abnormal shadow candidates having been detected, according to the result of comparison between the indicator value, the threshold value TH1 for detecting the falsely positive candidate, and the threshold value TH2 for detecting the truly positive candidate. The other abnormal shadow candidates other than these are extracted as the abnormal shadow candidates to be displayed.

For example, the following describes the conditions wherein, assuming that threshold value TH1 = 0.25 and threshold value TH2 = 0.85, the candidates having an indicator value lower than the TH1 are falsely positive, and the candidates having an indicator value higher than the TH2 are truly positive. The indicator value for the truly positive property outputted at the time of detection (hereinafter referred to as "S") is outputted as 0 through 1 (the possibility of being truly positive is lower as the value is closer to 0, and the possibility of being truly positive is higher as the value is closer to 1). Then the abnormal shadow candidates having the indicator S being in the relationship of TH1 < S < TH2 is extracted out of the detected candidates.

To be more specific, the extracted candidates are extracted from the abnormal shadow candidates detected by the process of detection to be the candidates except for clearly falsely positive and truly positive ones, in other words, the extracted candidates are the candidates which are doubtful whether they are truly positive or not, and the final decision should be left to the doctor. The following arrangement can also be used: The abnormal shadow candidates in the breast region close to the edge of the image tend to be overlooked by the doctor by its very nature of being close to the edge. When the abnormal shadow candidates located at the position likely to be overlooked by the doctor are wanted to be displayed as well, the distance from the edge of the image is obtained in advance as a boundary for the region which is difficult to find, and the value having been obtained is assumed as threshold value TH3. Of the abnormal shadow candidates having been detected, the ones having a value below the threshold value TH3 (located close to the image edge) are extracted as the abnormal shadow candidates to be displayed.

It should be noted that the threshold values TH1 and TH2 used to determine whether they are truly positive or falsely positive can be set for each radiographic interpretation. To put it more specifically, arrangements are made in such a way that setting information of the threshold values TH1 and TH2 can be inputted through the operation section 22, and setting information of threshold values TH1 and TH2 is stored in the storing section 25 after being associated with the ID of each doctor interpreting a radiograph. The ID of the doctor is inputted at the time of radiographic interpretation. Then the threshold values TH1 and TH2 corresponding to the doctor is read by the control section 21 and is used for the aforementioned processing.

If a doctor wishes to minimize the number of the falsely positive shadow candidates to be displayed, the setting of the threshold value TH1 is changed from 0.25 to 0.3. Thus, the number of the abnormal shadow candidates to be displayed can be adjusted by setting the threshold value TH1 or TH2 to conform to the skill and interpreting style of the doctor. This arrangement provides the optimum display best suited to the skill of each doctor.

In the manner discussed above, the abnormal shadow candidates to be displayed are extracted. Then the detection information of the abnormal shadow candidates having been extracted is displayed on the display section 23 (Step T34).

Fig. 18 shows an example of displaying the detection information of the abnormal shadow candidates using the processing patterns 1 through 3.

Fig. 18 (a) shows an example of display using the pattern 1 or 2. Fig. 18 (b) shows an example of display using the pattern 3. In Figs. 18 (a) and (b), a marker image showing the detection position (indicated by an arrow in the diagram) is shown as the detection information of the abnormal shadow candidates on the breast image.

In the processing pattern 1 or 2, only the candidates having a higher possibility of being truly positive are outputted as a result of detection. This makes it possible to display only the shadows having a higher possibility of being abnormal, as shown in Fig. 18 (a).

In the processing pattern 3, in the meantime, only the candidates other than those having a higher possibility of being truly positive or falsely positive are outputted as a result of detection. This makes it possible to show only the markers for the shadows wherein it is difficult to determine from the shape and density whether they are truly positive or falsely positive.

As described above, according to the present embodiment, when the processes of detection containing different processing patterns 1 through 3 are implemented by a combination of two processes of detection, the candidates whose detection information is to be displayed is extracted from the detected abnormal shadow candidates in conformity to the process of detection, and the detection information is displayed only for the abnormal shadow candidates having been extracted. This arrangement ensures that the number of items of the detection information to be displayed is reduced in conformity to the process of detection, whereby easy-to-read display is provided, and the efficiency of the radiographic interpretation by the doctor is enhanced.

For the processing pattern 1 or 2 with a higher percentage of detecting the truly positive candidates, all the abnormal shadow candidates are extracted. For the processing pattern 3 with a higher percentage of detecting the falsely positive candidates, the extracted candidates are those wherein it is difficult to determine whether they are truly positive or not, and the candidates of low visibility which tend to be overlooked by a doctor. In conformity to the difference in the percentage of detection that may occur in the process of detection, the number of the items of detection information of the abnormal shadow candidates is changed. This makes it possible to reduce the number of display items.

(Another embodiment of the present invention)

The following describes another embodiment of the present invention with reference to the operation of the aforementioned medical image processing system 100. The present embodiment includes a plurality of detection algorithms conforming to the type of the lesion to be detected. When abnormal shadow candidates are detected using a plurality of detection algorithms conforming to the type of the lesion to be detected, the abnormal shadow candidates to be displayed are extracted in conformity to different detection processes of the detection algorithm employed, and the detection information of the extracted abnormal shadow candidates is displayed and outputted. This will be taken as an example in the following description.

In the present embodiment, the storing section 25 of the image processing apparatus 2 incorporates a detection processing program using the detection algorithm in conformity to the type of the lesion to be detected. In the present embodiment, it stores the detection algorithm programs conforming to the two types of lesions, namely tumor and micro-calcification clusters as lesions in the breast.

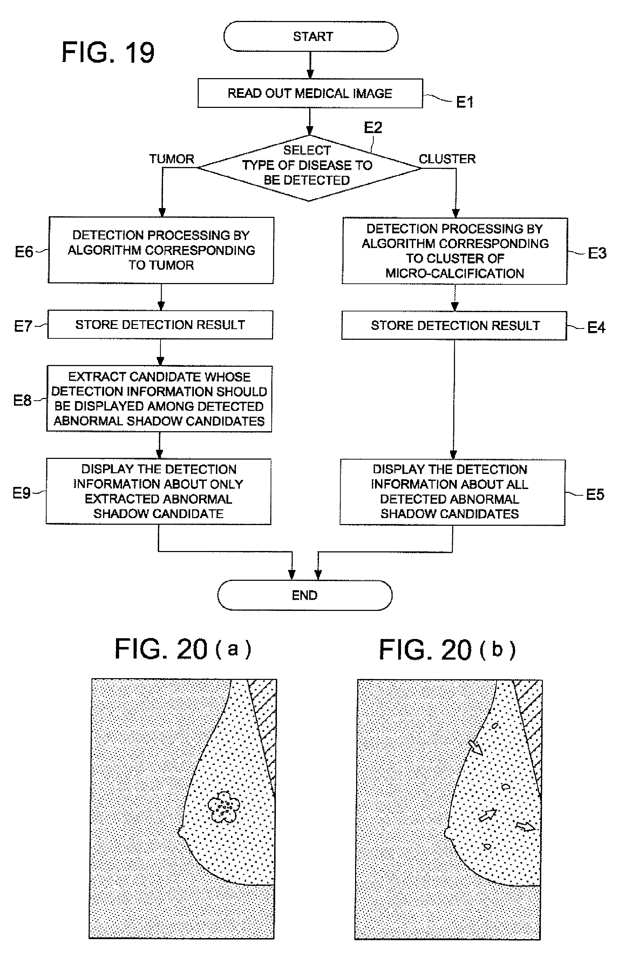

Referring to Fig. 19, the following describes the processing of detecting the abnormal shadow candidates of the image processing apparatus 2 in the present embodiment:

In the process of detecting the abnormal shadow candidates in Fig. 19, a medical image to be processed is read out from the storing section 25 by the abnormal shadow candidate detecting section 27 (Step E1). This is followed by the step of displaying the selection screen in the display section 23 to select the type of lesion to be detected. Guidance is given to prompt selection of any one of the tumor and micro-calcification clusters whose abnormal shadow candidates are to be detected.

Then any one of the lesions is selected by the doctor on the selection screen through the operation section 22 (Step E2). If the micro-calcification cluster has been selected (Step E2: Cluster), the system goes to Step E3. If the tumor has been selected (Step E2: Tumor), the system goes to Step E6.

The following describes the case when the micro-calcification cluster has been selected.

In Step E3, the detection processing program conforming to the micro-calcification cluster is read from the storing section 25 in the abnormal shadow candidate detecting section 27, and the abnormal shadow candidates are detected through collaboration with this program. To be more specific, detection is made by the detection algorithm conforming to the detection of the micro-calcification cluster.

In the detection algorithm of the micro-calcification cluster, the candidate region is specified by a triple ring filter or others. The step of detection is carried out (image processing 2) using the characteristic amount. The details of processing have already been discribed, and will not described here.

Upon completion of all the steps of detection, the information on the position and area of the abnormal shadow candidates having been detected is stored in the storing section 25 as a result of detecting the micro-calcification cluster (Step E4). Then information on the detection result is read from the storing section 25 by the control section 21, and the abnormal shadow candidate extraction conditions preset for the step of detecting the micro-calcification cluster is read out. The abnormal shadow candidate extraction conditions preset for the step of detecting the micro-calcification cluster represent extraction of all abnormal shadow candidates. To be more specific, all the detected abnormal shadow candidates are extracted as the candidates to be displayed, based on the information having been read out. The detection information of all the detected abnormal shadow candidates is displayed on the display section 23 (Step E5).

The following describes the case wherein the tumor is selected.

In Step E6, in the abnormal shadow candidate detecting section 27, a detection processing program in conformity to the tumor is read from the storing section 25, and the abnormal shadow candidates are detected through collaboration with this program. To be more specific, detection is made by the detection algorithm conforming to the detection of the tumor.

In the tumor detection algorithm, a step of detection in processing 1 using a filter bank is carried out.

Upon completion of all the steps of detection, the information on the position and area of the detected abnormal shadow candidates is stored in the storing section 25 as a result of detection (Step E7). This is followed by the step of the information on the result of detection being read from the storing section 25 by the control section 21. Of the abnormal shadow candidates having been detected, the candidates whose detection information is to be displayed are extracted (Step E8).

In the case of the micro-calcification cluster, the shadow appears on the screen as a collection (cluster) of fine white spots having density variations of inverted conical structure. Thus, a marker image of the frame enclosing the portion wherein fine shadows are clustered is outputted as the detection information of the detected abnormal shadow candidates. To be more specific, when there are a great number of falsely positive candidates, there is an increase in the set area. Since the shadow is very small, the amount of change is small. Thus, even if there are a great number of detected abnormal shadow candidates, only the size of the frame is subjected to change, without the visibility being much affected.

In the case of a tumor, however, an arrow or the like that indicates each candidate of the detected tumor is used as a marker. If there are a great number of candidates, the display area is occupied by them, and this may adversely affect radiographic interpretation. Thus, of the abnormal shadow candidates having been detected, those to be displayed are extracted, whereby the number of the detection information items to be displayed is reduced.

In the case of a tumor, a clearly truly positive shadow has its size of about 3 through 5 mm and is larger than the micro-calcification shadow. The outline on the margin of the lesion (difference in density from the surrounding tissue) is clear (still clear due to edge effect in the case of phase contrast radiographing), and the shadow can be identified by visual observation of the doctor. Accordingly, similarly to the case of the first embodiment, the control section 21 extracts the candidates wherein it is difficult to determine whether they are truly or falsely positive and the final decision should be left to the doctor, and the candidates of low visibility in the detected position which tend to be overlooked by a doctor. The extraction method will not be described here because it is the same in the abovementioned embodiment.

When the abnormal shadow candidates to be displayed have been extracted in the manner discussed above, the detection information of the extracted abnormal shadow candidates is displayed on the display section 23 (Step E8).

Fig. 20 shows an example of detection information of abnormal shadow candidates.

Fig. 20 (a) is a diagram showing an example of displaying a micro-calcification cluster candidate. Fig. 20 (b) is a diagram showing an example of displaying a tumor candidate. In the case of the micro-calcification cluster, a marker of a frame enclosing the collection of the candidates is as shown in Fig. 20 (a) to ensure that the doctor can identify the set of the shadows of the micro-calcified portion appearing in dots. For convenience, though the shadow of micro-calcification cluster is shown in black dots, actually it appears as whitish shadows of low fat. In the meantime, the tumor having a certain size of 3 through 5 mm, for example, appears on the image, and therefore, an arrow indicating the position of detection is used as the marker of the tumor candidate as shown in Fig. 20 (b).

Even when a great number of falsely positive candidates have been detected, the micro-calcification cluster is very likely to be detected around the truly positive candidates and even when there are a great number of falsely positive candidates, there is a slight variation in the region wherein the calcified portions are assembled. Thus, even if detection information has been displayed for all the candidates having been detected, there is a change in only the size of the frame as a marker as shown in Fig. 20 (a), without the number of frames being increased very much. This does not disturb the display of detection information at the time of interpretation of the medical image.

In the meantime, in the case of a tumor, a marker is shown for one candidate. If there are a great number of falsely positive candidates, there is an increase in the number of markers of the detection information on the medical image, with the result that the display is hard to see.

Thus, a marker is displayed only for the shadows wherein the shape or density cannot easily determine whether the candidates are truly positive or falsely positive, so that the number of displayed items is reduced. This arrangement provides the form of display that facilitates radiographic interpretation.

It is also possible to arrange such a configuration that the display of the micro-calcification cluster shown in Fig. 20 (a) and the display of the tumor of Fig. 20 (b) are switched in response to the operation by the doctor. It is also possible to use the method of display on one and the same breast image.

As described above, according to the present embodiment, when different steps of detection are used in response to the object of detection, the candidates whose detection information is to be displayed is extracted from the abnormal shadow candidates detected according to the step of detection. The detection information is displayed only for the abnormal shadow candidates having been extracted. This arrangement reduces detection information to be displayed, in response to the step of detection, whereby an easy-to-read display can be provided and efficiency in radiographic interpretation by the doctor can be enhanced.

All the candidates having been detected are extracted in the case of the micro-calcification cluster wherein the set of the candidates detected is enclosed by the frame as detection information. In the case of the tumor wherein an arrow is used to indicate the detection position of each candidate, the candidates wherein it is difficult to determine whether they are truly or falsely positive, and the candidates of low visibility which tend to be overlooked by a doctor are extracted. The number of displayed detection information items is changed in response to the difference in the percentage of detection resulting from such a detection step. This arrangement allows the number of displays to be adjusted to be reduced.

Such functions as detection of the abnormal shadow candidates and extraction of the candidates to be displayed have been described as being implemented by the image processing apparatus 2. Without the present invention being restricted thereto, these functions can be implemented by each component of the medical image processing system 100 such as a viewer 5. Alternatively, a special-purpose apparatus can be installed to perform these functions.

In the above description, reference has been made to an example wherein the detection information of the abnormal shadow candidates is displayed and outputted by the viewer 5. Without the present invention being restricted thereto, the present invention applies to the cases wherein a printer 3 is used for film output.

**Claims**

1.  An abnormal shadow candidate display method including the steps of:

    detecting an abnormal shadow candidate by analyzing an medical image;
    extracting an abnormal shadow candidate to be displayed among abnormal shadow candidates having been detected; and
    displaying a detection information on the abnormal shadow candidate having been extracted.

2.  The abnormal shadow candidate display method described in claim 1,
    wherein the extracting step includes the steps of:

    calculating a characteristic amount of an image of the abnormal shadow candidate having been detected; and
    determining a priority order of the abnormal shadow candidate to be displayed according to the characteristic amount of an image,

    wherein an abnormal shadow candidate of the higher priority is extracted on a priority basis among the detected abnormal shadow candidates.

3.  The abnormal shadow candidate display method described in claim 2,
    wherein, in the extracting step, a contrast between the abnormal shadow candidate and a surrounding region thereof is calculated as the characteristic amount of an image, and the higher priority is given to an abnormal shadow candidate of smaller contrast.

4.  The abnormal shadow candidate display method described in claim 2,
    wherein, in the extracting step, an area of a region of the abnormal shadow candidate is calculated as the characteristic amount of an image, and the higher priority is given to an abnormal shadow candidate of smaller area.

5.  The abnormal shadow candidate display method described in claim 2,
    wherein, in the extracting step, a distance from an edge of the medical image to a detected position of the abnormal shadow candidate is calculated as the characteristic amount of an image, and the higher priority is given to an abnormal shadow candidate of shorter distance.

6.  The abnormal shadow candidate display method described in any one of claims 2 - 5, further comprising the steps of identifying a subject region from the medical image; and
    classifying the subject region into a plurality of regions,
    wherein, in the extracting step, the priority order is determined according to the classified region in which the abnormal shadow candidate has been detected among subject regions having been identified.

7.  The abnormal shadow candidate display method described in claim 2,
    wherein, in the extracting step, a characteristic amount of a shape of the abnormal shadow candidate is calculated as the characteristic amount of an image, and the priority order is determined according to the characteristic amount of a shape.

**8.** The abnormal shadow candidate display method described in claim 2,
wherein, in the extracting step, a density of a region of the abnormal shadow candidate is calculated as the characteristic amount of an image, and the higher priority is given to an abnormal shadow candidate having a characteristic amount of lower density.

**9.** The abnormal shadow candidate display method described in claim 2,
wherein, in the extracting step, at least one of an area of a region of the abnormal shadow candidate, density of a region of the abnormal shadow candidate, contrast to a surrounding region, a shape of the abnormal shadow candidate, and a distance from an edge of the medical image to a detected position of the abnormal shadow candidate is calculated as the characteristic amount of an image, and the priority order is determined according to the characteristic amount having been calculated.

**10.** The abnormal shadow candidate display method described in claim 1 or 2,
wherein, in the displaying step, the detection information of the abnormal shadow candidate having been extracted is displayed together with the medical image.

**11.** The abnormal shadow candidate display method described in claim 10,
wherein the medical image is captured by a phase contrast radiographing method.

**12.** The abnormal shadow candidate display method described in claim 10 or 11,
wherein, in the displaying step, a life-size medical image obtained by reducing the medical image to a same size as that of a subject is displayed.

**13.** The abnormal shadow candidate display method described in claim 12,
wherein a reference image is created from the medical image and an image obtained by superimposing the detection information on the created image is displayed together with the life-size image.

**14.** The abnormal shadow candidate display method described in claim 1,
wherein, in the extracting step, the abnormal shadow candidate to be displayed is determined and extracted, among the abnormal shadow candidates having been detected, based on a threshold value preset to determine whether the candidate is to be displayed or not.

**15.** The abnormal shadow candidate display method described in claim 14,
wherein the threshold value includes a first threshold value and a second threshold value and, in the extracting step, the abnormal shadow candidate to be displayed is determined based on the first and the second threshold values and is extracted among the abnormal shadow candidates having been detected.

**16.** The abnormal shadow candidate display method described in claim 15,
wherein the first threshold value is a threshold value to delete a falsely positive candidate and the second threshold value is a threshold value to delete a truly positive candidate, and in the extracting step, an abnormal shadow candidate remaining after deletion of the falsely positive candidate and the truly positive candidate from the abnormal shadow candidates having been detected by using the first and the second threshold values is extracted to be displayed.

**17.** The abnormal shadow candidate display method described in claim 15 or 16,
wherein the first or the second threshold value can be set for each doctor interpreting a radiograph.

**18.** The abnormal shadow candidate display method described in claim 15 or 16,
wherein the first threshold value is set commonly for all the doctors interpreting a radiograph, and the second threshold value can be set for each doctor interpreting a radiograph.

**19.** The abnormal shadow candidate display method described in any one of claims 14 - 18,
wherein the threshold value contains a third threshold value for determining whether visibility on the image is low or not, and in the extracting step, the abnormal shadow candidate having been determined by the third threshold value as having low visibility is extracted from the detected abnormal shadow candidates.

**20.** A medical image processing system comprising:

an abnormal shadow candidate detecting device for analyzing an medical image and detecting an abnormal shadow candidate;

a control device for extracting an abnormal shadow candidate to be displayed, among abnormal shadow candidates having been detected by the abnormal shadow candidate detecting device; and

a display device for displaying detection information on the abnormal shadow candidate having been extracted.

21. The medical image processing system described in claim 20,
wherein the display device displays the detection information on the extracted abnormal shadow candidates together with the medical image.

22. The medical image processing system described in claim 21,
wherein the medical image is a medical image captured by a phase contrast radiographing method.

23. The medical image processing system described in claim 20,
wherein the control device determines and extracts the abnormal shadow candidate to be displayed, among the abnormal shadow candidates having been detected, based on a threshold value preset to determine whether the candidate is to be displayed or not.

# FIG. 1

EP 1 884 193 A1

100

```
                    1                    4          4a              5
           ┌──────────────┐      ┌──────────┐   ┌────────┐   ┌──────────┐
           │    IMAGE     │      │  IMAGE   │   │ IMAGE  │   │  VIEWER  │
           │  GENERATING  │      │  SERVER  │───│   DB   │   │          │
           │  APPARATUS   │      │          │   │        │   │          │
           └──────────────┘      └──────────┘   └────────┘   └──────────┘
                  │                    │                          │
       ───────────┴──────────┬────────┴──────────┬───────────────┴──────── N
                             │                    │
                        ┌──────────┐         ┌──────────┐
                    2   │  IMAGE   │     3   │ PRINTER  │
                        │PROCESSING│         │          │
                        │APPARATUS │         │          │
                        └──────────┘         └──────────┘
```

# FIG. 2

IMAGING POSITION AT NORMAL RADIOGRAPHING METHOD

IMAGING POSITION AT PHASE CONTRAST RADIOGRAPHING METHOD

X-RAY IMAGE DETECTOR

X-RAY IMAGE DETECTOR

X-RAY SOURCE

SUBJECT

CONTACT IMAGE

ENLARGED IMAGE

EP 1 884 193 A1

# FIG. 3

EP 1 884 193 A1

# FIG. 4

2

```
                    CONTROL
                    SECTION        ─ 21
```

```
22 ─  OPERATION              STORING SECTION  ─ 25
      SECTION
```

```
23 ─  DISPLAY SECTION        IMAGE
                             PROCESSING       ─ 26
                             SECTION
```

```
24 ─  COMMUNICATION          ABNORMAL SHADOW
      SECTION                CANDIDATE        ─ 27
                             DETECTING SECTION
```

# FIG. 5

# FIG. 6

START

EXTRACT ANATOMICAL AREA — S1

DETECT ABNORMAL SHADOW CANDIDATE — S2

DECIDE PRIORITY ORDER FOR DISPLAY BASED ON CHARACTERISTIC AMOUNT OF DETECTED ABNORMAL SHADOW CANDIDATE — S3

EXTRACT DETECTED ABNORMAL SHADOW CANDIDATE UP TO PRESCRIBED PRIORITY ORDER PREFERENTIALLY AND OUTPUT AS DETECTION RESULT — S4

END

# FIG. 7

```
┌─────────────────────────┐
│  DETECTION OF ABNORMAL  │
│    SHADOW CANDIDATE     │
└─────────────────────────┘
            │
            ▼
┌─────────────────────────┐
│   PRIMARY DETECTION     │────── S21
└─────────────────────────┘
            │
            ▼
┌─────────────────────────┐
│    CALCULATE IMAGE      │
│ CHARACTERISTIC AMOUNT   │
│ OF CANDIDATE OF PRIMARY │────── S22
│      DETECTION          │
└─────────────────────────┘
            │
            ▼
┌─────────────────────────┐
│  CONDUCT MULTIVARIATE   │
│    ANALYSIS BY USING    │
│ CHARACTERISTIC AMOUNT OF│
│ IMAGE AND OUTPUT INDICATOR│──── S23
│ VALUE OF ABNORMAL SHADOW│
└─────────────────────────┘
            │
            ▼
┌─────────────────────────┐
│JUDGE WHETHER TO REGARD IT AS│
│ ABNORMAL SHADOW CANDIDATE│
│OR NOT BASED ON THE INDICATOR│── S24
│ VALUE AND THRESHOLD VALUE│
└─────────────────────────┘
            │
            ▼
┌─────────────────────────┐
│     TO THE NEXT         │
│     PROCESSING          │
└─────────────────────────┘
```

# FIG. 8

STARTING
POINT

## FIG. 9 ( a )

## FIG. 9 ( b )

## FIG. 9 ( c )

G1

G2

g1

g2

# FIG. 10

```
                    ┌──────────────────┐
                    │      START       │
                    └──────────────────┘
                             │
                             ▼
        ┌────────────────────────────────┐
        │   EXTRACT ANATOMICAL AREA      │
        │     FROM MEDICAL IMAGE         │────── T1
        └────────────────────────────────┘
                             │
                             ▼
        ┌────────────────────────────────┐
        │  DETECT ABNORMAL SHADOW        │
        │  CANDIDATE IN THE EXTRACTED    │
        │  BREAST AREA → DETECTION       │────── T2
        │  CANDIDATES R1                 │
        └────────────────────────────────┘
                             │
                             ▼
        ┌────────────────────────────────┐
        │     CALCULATE IMAGE            │
        │  CHARACTERISTIC AMOUNT OF      │────── T3
        │  DETECTION CANDIDATES R1       │
        └────────────────────────────────┘
                             │
                             ▼
        ┌────────────────────────────────┐
        │  DELETE FALSE-POSITIVE         │
        │  CANDIDATE FROM DETECTION      │
        │  CANDIDATES R1 BY THRESHOLD    │
        │  VALUE TH1 BASED ON IMAGE      │────── T4
        │  CHARACTERISTIC AMOUNT         │
        │  → DETECTION CANDIDATES R2     │
        └────────────────────────────────┘
                             │
                             ▼
        ┌────────────────────────────────┐
        │  DELETE TRUE-POSITIVE          │
        │  CANDIDATE FROM DETECTION      │
        │  CANDIDATES R2 BY THRESHOLD    │
        │  VALUE TH2 BASED ON IMAGE      │────── T5
        │  CHARACTERISTIC AMOUNT         │
        │  → DETECTION CANDIDATES R3     │
        └────────────────────────────────┘
                             │
                             ▼
                    ┌──────────────────┐
                    │       END        │
                    └──────────────────┘
```

## FIG. 11 ( a )  FIG. 11 ( b )  FIG. 11 ( c )

FIG. 12

# FIG. 13

```
                  ┌──────────────┐
                  │    START     │
                  └──────┬───────┘
                         │
          ┌──────────────▼──────────────┐
          │   READ OUT MEDICAL IMAGE    │──── S31
          └──────────────┬──────────────┘
                         │
          ┌──────────────▼──────────────┐
          │ ABNORMAL SHADOW CANDIDATE   │
          │   DETECTION PROCESSING      │
          │    BY USING FILTER BANK     │──── S32
          │      (PROCESSING 1)         │
          └──────────────┬──────────────┘
                         │
          ┌──────────────▼──────────────┐
          │   STORE DETECTION RESULT    │──── S33
          │      OF PROCESSING 1        │
          └──────────────┬──────────────┘
                         │
          ┌──────────────▼──────────────┐
          │   READ OUT MEDICAL IMAGE    │──── S34
          └──────────────┬──────────────┘
                         │
          ┌──────────────▼──────────────┐
          │   PRE-PROCESSING BASED ON   │
          │    DETECTION RESULT OF      │──── S35
          │        PROCESSING 1         │
          └──────────────┬──────────────┘
                         │
          ┌──────────────▼──────────────┐
          │    ABNORMAL SHADOW          │
          │  CANDIDATE DETECTION        │
          │  PROCESSING BY USING        │──── S36
          │  CHARACTERISTIC AMOUNT      │
          │     (PROCESSING 2)          │
          └──────────────┬──────────────┘
                         │
          ┌──────────────▼──────────────┐
          │   STORE DETECTION RESULT    │──── S37
          │      OF PROCESSING 2        │
          └──────────────┬──────────────┘
                         │
          ┌──────────────▼──────────────┐
          │ DISPLAY DETECTION INFORMATION│
          │ ABOUT ALL ABNORMAL SHADOW   │
          │  CANDIDATES DETECTED BY     │──── S38
          │        PROCESSING 2         │
          └──────────────┬──────────────┘
                         │
                  ┌──────▼───────┐
                  │     END      │
                  └──────────────┘
```

# FIG. 14

BAND DIVISION FILTER BANK

BAND COMPOSITION FILTER BANK

$A(z^j) =$

$S_{j-1}f \rightarrow$ $H_L(z^j) F_L(z^j) \rightarrow H_L(z^j) F_L(z^j) \rightarrow S_jf$

$H_H(z^j) F_H(z^j) \rightarrow Wh_jf$

$H_H(z^j) F_H(z^j) \rightarrow Wv_jf$

$H_H(z^j) \rightarrow H_H(z^j) \rightarrow Wd_jf$

HORIZONTAL DIRECTION | VERTICAL DIRECTION

$S(z^j) =$

$S_jf \rightarrow S_{j-1}f$

$Wh_jf \rightarrow H_L(z^j) F_L(z^j)$

$Wv_jf \rightarrow H_L(z^j) F_L(z^j)$

$Wd_jf \rightarrow F_H(z^j) \rightarrow F_H(z^j)$

VERTICAL DIRECTION | HORIZONTAL DIRECTION

## FIG. 15

```
┌─────────────────────────────────┐
│   ABNORMAL SHADOW CANDIDATE      │
│   DETECTION PROCESSING           │
│   BY USING FILTER BANK (PROCESSING 1) │
└─────────────────────────────────┘
                │
                ▼
┌─────────────────────────────────┐
│  INPUT IMAGE DATA TO FILTER BANK │──── S41
└─────────────────────────────────┘
                │
                ▼
┌─────────────────────────────────┐
│  CALCULATE MAXIMUM AMD MINIMUM   │
│  EIGENVALUES OF HESSIAN MATRIX   │──── S42
│  FROM IMAGES OF PLURAL LEVELS    │
│  OF RESOLUTION                   │
└─────────────────────────────────┘
                │
                ▼
┌─────────────────────────────────┐
│  GENERATE ENHANCED IMAGE         │──── S43
└─────────────────────────────────┘
                │
                ▼
┌─────────────────────────────────┐
│  SET REGION OF INTEREST AND CALCULATE │
│  CHARACTERISTIC AMOUNT OF CIRCULAR    │──── S44
│  AND LINEAR COMPONENTS IN THE REGION  │
└─────────────────────────────────┘
                │
                ▼
┌─────────────────────────────────┐
│  DISCRIMINATE ABNORMAL SHADOW BASED │
│  ON THE CHARACTERISTIC AMOUNT       │──── S45
└─────────────────────────────────┘
                │
                ▼
          ┌───────────┐
          │    END    │
          └───────────┘
```

## FIG. 16

```
┌─────────────────────────────────┐
│   ABNORMAL SHADOW CANDIDATE      │
│   DETECTION PROCESSING BY USING  │
│   CHARACTERISTIC AMOUNT (PROCESSING 2) │
└─────────────────────────────────┘
                │
                ▼
┌─────────────────────────────────┐
│  SPECIFY SHADOW OR REGION INCLUDED │
│  IN IMAGE DATA                     │──── S51
└─────────────────────────────────┘
                │
                ▼
┌─────────────────────────────────┐
│  CALCULATE CHARACTERISTIC AMOUNT  │
│  IN SHADOW OR REGION              │──── S52
└─────────────────────────────────┘
                │
                ▼
┌─────────────────────────────────┐
│  DISCRIMINATE ABNORMAL SHADOW BASED │
│  ON THE CHARACTERISTIC AMOUNT       │──── S53
└─────────────────────────────────┘
                │
                ▼
          ┌───────────┐
          │    END    │
          └───────────┘
```

# FIG. 17

```
              ┌─────────────┐
              │    START     │
              └──────┬──────┘
                     │
        ┌────────────▼────────────┐
        │  READ OUT MEDICAL IMAGE  │──── T11
        └─────┬──────────────┬────┘
              │              │
   ┌──────────▼──────┐  ┌────▼──────────────┐
   │ ABNORMAL SHADOW  │  │ ABNORMAL SHADOW   │
   │ CANDIDATE        │  │ CANDIDATE         │
T21│ DETECTION        │  │ DETECTION         │T22
   │ PROCESSING       │  │ PROCESSING        │
   │ BY USING FILTER  │  │ BY USING          │
   │ BANK             │  │ CHARACTERISTIC    │
   │ (PROCESSING 1)   │  │ AMOUNT            │
   └──────────┬──────┘  │ (PROCESSING 2)    │
              │         └────┬──────────────┘
   ┌──────────▼──────┐  ┌────▼──────────────┐
T31│ STORE DETECTION │  │ STORE DETECTION   │T32
   │ RESULT OF       │  │ RESULT OF         │
   │ PROCESSING 1    │  │ PROCESSING 2      │
   └──────────┬──────┘  └────┬──────────────┘
              │              │
        ┌─────▼──────────────▼─────┐
        │ EXTRACT CANDIDATE WHOSE   │
        │ DETECTION INFORMATION     │
        │ SHOULD BE DISPLAYED AMONG │──── T33
        │ ABNORMAL SHADOW CANDIDATES│
        │ DETECTED IN PROCESSING    │
        │ 1 AND 2                   │
        └─────────────┬────────────┘
        ┌─────────────▼────────────┐
        │ DISPLAY THE DETECTION     │
        │ INFORMATION ONLY FOR      │──── T34
        │ EXTRACTED ABNORMAL        │
        │ SHADOW CANDIDATE          │
        └─────────────┬────────────┘
              ┌───────▼──────┐
              │     END      │
              └─────────────┘
```

# FIG. 18 ( a )          FIG. 18 ( b )

## FIG. 19

START

READ OUT MEDICAL IMAGE — E1

E2

SELECT TYPE OF DISEASE TO BE DETECTED

TUMOR / CLUSTER

E6 — DETECTION PROCESSING BY ALGORITHM CORRESPONDING TO TUMOR

DETECTION PROCESSING BY ALGORITHM CORRESPONDING TO CLUSTER OF MICRO-CALCIFICATION — E3

E7 — STORE DETECTION RESULT

STORE DETECTION RESULT — E4

E8 — EXTRACT CANDIDATE WHOSE DETECTION INFORMATION SHOULD BE DISPLAYED AMONG DETECTED ABNORMAL SHADOW CANDIDATES

E9 — DISPLAY THE DETECTION INFORMATION ABOUT ONLY EXTRACTED ABNORMAL SHADOW CANDIDATE

DISPLAY THE DETECTION INFORMATION ABOUT ALL DETECTED ABNORMAL SHADOW CANDIDATES — E5

END

## FIG. 20 (a)

## FIG. 20 (b)

## INTERNATIONAL SEARCH REPORT

| International application No. |
|---|
| PCT/JP2006/309278 |

A. CLASSIFICATION OF SUBJECT MATTER
***A61B6/00***(2006.01), ***A61B5/00***(2006.01), ***A61B6/03***(2006.01), ***G06T1/00***
(2006.01)

According to International Patent Classification (IPC) or to both national classification and IPC

B. FIELDS SEARCHED

Minimum documentation searched (classification system followed by classification symbols)
A61B6/00, A61B5/00, A61B6/03, G06T1/00

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br><br>A | JP 2003-299645 A (Hitachi Medical Corp.),<br>21 October, 2003 (21.10.03),<br>Full text; all drawings<br>(Family: none) | 1,2,10,20,21<br>3,4,6-9,<br>11-18,22,23<br>5,19 |
| Y<br><br>A | JP 2005-102784 A (Konica Minolta Medical &<br>Graphic, Inc.),<br>21 April, 2005 (21.04.05),<br>Full text; all drawings<br>(Family: none) | 3,4,7,9<br>5,19 |
| Y | JP 2004-180987 A (Hitachi Medical Corp.),<br>02 July, 2004 (02.07.04),<br>Par. No. [0016]<br>& US 2004/0120561 A1  & US 2003/0179915 A1 | 6 |

| ☒ | Further documents are listed in the continuation of Box C. | ☐ | See patent family annex. |

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>25 May, 2006 (25.05.06) | Date of mailing of the international search report<br>06 June, 2006 (06.06.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2006/309278 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2005-95501 A (Konica Minolta Medical & Graphic, Inc.), 14 April, 2005 (14.04.05), Full text; all drawings & US 2005/0069184 A1 & US 2005/0069184 A1 | 8,14-18,23 |
| Y | JP 2002-162705 A (Konica Corp.), 07 June, 2002 (07.06.02), Par. No. [0126] (Family: none) | 11-13,22 |
| Y | JP 2005-124617 A (Konica Minolta Medical & Graphic, Inc.), 19 May, 2005 (19.05.05), Full text; all drawings & US 2004/0151358 A1 | 13 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000276587 A
- JP 2004230001 A
- JP 2001238868 A
- JP H1091758 B

- JP 2001238871 A
- JP 2001311701 A
- JP 2002085389 A
- JP 2001299733 A


**Non-patent literature cited in the description**

- *Collected Research Papers of the Institute of Electronics, Information and Communication Engineers (D-II,* 1993, vol. J76-D-II (2), 241-249
- *Collected Research Papers of the Institute of Electronics, Information and Communication Engineers (D-II,* 1992, vol. J71-D-II (7), 1170-1176
- *Collected Research Papers of the Institute of Electronics, Information and Communication Engineers (D-II,* 1998, vol. J71-D-II (10), 1994-2001
- *Collected Research Papers of the Institute of Electronics, Information and Communication Engineers D-II,* vol. J87-D-II (1), 186-196

- *Collected Research Papers of the Institute of Electronics, Information and Communication Engineers D-II,* 1993, vol. J76-D-II (2), 241-249
- *Collected Research Papers of the Institute of Electronics, Information and Communication Engineers D-II,* 1992, vol. J71-D-II (7), 1170-1176
- *Collected Research Papers of the Institute of Electronics, Information and Communication Engineers D-II,* 1998, vol. J71-D-II (10), 1994-2001